# EUROPÄISCHE PATENTSCHRIFT

(11) **EP 1 526 867 B1**
(45) Veröffentlichungstag und Bekanntmachung des Hinweises auf die Patenterteilung: **25.04.2007**
(21) Anmeldenummer: 03766302.8
(22) Anmeldetag: 25.07.2003
(51) Int. Cl.: A61K 38/18, A61P 9/00, A61P 17/02

(54) **ERYTHROPOIETIN IN SUBPOLYCYTHÄMISCHEN DOSEN**
ERYTHROPOIETIN IN SUBPOLYCYTHEMIC DOSES
ERYTHROPOIETINE A DOSES SUBPOLYCYTHEMIQUES

(30) Priorität: 26.07.2002 DE 10234192
(43) Veröffentlichungstag der Anmeldung: 04.05.2005
(60) Teilanmeldung: 07001689.4
(73) Patentinhaber: EPOPLUS GmbH & Co. KG, 30625 Hannover (DE)
(72) Erfinder: BAHLMANN, Ferdinand, Hermann, 30173 Hannover (DE); HALLER, Hermann, 30559 Hannover (DE)
(74) Vertreter: Schrell, Andreas
(86) Internationale Anmeldenummer: PCT/EP2003/008229
(87) Internationale Veröffentlichungsnummer: WO 2004/012759

(56) Entgegenhaltungen:
- WO-A-00/61164
- WO-A-02/14356
- WO-A-89/07944
- WO-A-92/15323
- WO-A-98/10650
- WO-A-02/085940
- WO-A-03/037273
- WO-A-03/057242
- US-A- 4 992 419
- US-A- 5 198 417
- US-A- 5 980 887
- US-A1- 2002 065 214
- BUEMI M ET AL: "Recombinant erythropoietin prevents the progression of atherosclerosis in Watanabe rabbits with hereditary hypocholesterolemia" NEPHROLOGY DIALYSIS TRANSPLANTATION, Bd. 12, Nr. 9, 1997, Seite A190, XP0009021639 & ANNUAL CONGRESS OF THE EUROPEAN RENAL ASSOCIATION AND THE EUROPEAN DIALYSIS AND TRANSPLANT ASSOCIATI; GENEVA, SWITZERLAND; SEPTEMBER 21-24, 1997 ISSN: 0931-0509
- BUEMI M ET AL: "Recombinant human erythropoietin (rHuEPO): More than just the correction of uremic anemia" JOURNAL OF NEPHROLOGY 2002 ITALY, Bd. 15, Nr. 2, 2002, Seiten 97-103, XP0009021789 ISSN: 1121-8428
- MASUDA SEIJI ET AL: "Erythropoietic, neurotrophic, and angiogenic functions of erythropoietin and regulation of erythropoietin production" INTERNATIONAL JOURNAL OF HEMATOLOGY, Bd. 70, Nr. 1, Juli 1999 (1999-07), Seiten 1-6, XP0009021621 & ISSN: 0925-5710
- KRAUSE K ET AL: "Recombinant human erythropoietin and VEGF have equal angiogenic potency: Investigation in a novel in vitro assay of human vascular tissues" EUROPEAN HEART JOURNAL, Bd. 22, Nr. Abstract Supplement, September 2001 (2001-09), Seite 154, XP0001097312 & XXIII CONGRESS OF THE EUROPEAN SOCIETY OF CARDIOLOGY TOGETHER WITH THE 36TH ANNUAL GENERAL MEETING O; STOCKHOLM, SWEDEN; SEPTEMBER 01-05, 2001 ISSN: 0195-668X
- MASCHIO G: "Erythropoietin and systemic hypertension" NEPHROLOGY DIALYSIS TRANSPLANTATION, Bd. 10, Nr. SUPPL. 2, 1995, Seiten 74-79, XP0009021640 & ISSN: 0931-0509
- DATABASE BIOSIS [Online] BIOSCIENCES INFORMATION SERVICE, PHILADELPHIA, PA, US; November 2002 (2002-11), KASHIWAGI M ET AL: "Hypertension in a pregnancy with renal anemia after recombinant human erythropoietin (rhEPO) therapy." XP002275413 Database accession no. PREV200300039039 & ARCHIVES OF GYNECOLOGY AND OBSTETRICS, Bd. 267, Nr. 1, November 2002 (2002-11), Seiten 54-56, ISSN: 0932-0067
- DATABASE BIOSIS [Online] BIOSCIENCES INFORMATION SERVICE, PHILADELPHIA, PA, US; 1997, CONRAD KIRK P ET AL: "Placental cytokines and the pathogenesis of preeclampsia" XP002275414 Database accession no. PREV199799525116 & AMERICAN JOURNAL OF REPRODUCTIVE IMMUNOLOGY, Bd. 37, Nr. 3, 1997, Seiten 240-249, ISSN: 1046-7408
- DATABASE EMBASE [Online] ELSEVIER SCIENCE PUBLISHERS, AMSTERDAM, NL; 2000, CASES A: "Recombinant human erythropoietin treatment in chronic renal failure: Effects on hemostasis and vasculature" XP002275415 Database accession no. EMB-2000364537 & DRUGS OF TODAY 2000 SPAIN, Bd. 36, Nr. 8, 2000, Seiten 541-556, ISSN: 0025-7656
- DATABASE MEDLINE [Online] US NATIONAL LIBRARY OF MEDICINE (NLM), BETHESDA, MD, US; Mai 1996 (1996-05), BRAGA J ET AL: "Maternal and perinatal implications of the use of human recombinant erythropoietin." XP002275416 Database accession no. NLM8677769 & ACTA OBSTETRICIA ET GYNECOLOGICA SCANDINAVICA. MAY 1996, Bd. 75, Nr. 5, Mai 1996 (1996-05), Seiten 449-3, ISSN: 0001-6349
- DATABASE BIOSIS [Online] BIOSCIENCES INFORMATION SERVICE, PHILADELPHIA, PA, US; 16. November 2001 (2001-11-16), ARCASOY MURAT O ET AL: "Erythropoietin (EPO) stimulates angiogenesis in vivo and promotes wound healing" XP002275417 Database accession no. PREV200200261552 & BLOOD, Bd. 98, Nr. 11 Part 1, 16. November 2001 (2001-11-16), Seiten 822a-823a, 43RD ANNUAL MEETING OF THE AMERICAN SOCIETY OF HEMATOLOGY, PART 1; ORLANDO, FLORIDA, USA; DECEMBER 07-11, 2001 ISSN: 0006-4971
- DATABASE BIOSIS [Online] BIOSCIENCES INFORMATION SERVICE, PHILADELPHIA, PA, US; November 1998 (1998-11), ALVAREZ ARROYO MARIA VICTORIA ET AL: "Role of vascular endothelial growth factor on erythropoietin-related endothelial cell proliferation" XP002275418 Database accession no. PREV199800506855 & JOURNAL OF THE AMERICAN SOCIETY OF NEPHROLOGY, Bd. 9, Nr. 11, November 1998 (1998-11), Seiten 1998-2004, ISSN: 1046-6673

## Beschreibung

Die vorliegende Erfindung betrifft die Verwendung von Erythropoietin zur Herstellung einer pharmazeutischen Zusammensetzung enthaltend eine subpolycythämische EPO-Dosis, die einer wöchentlichen Dosis von 1 bis 90 IU EPO/kg Körpergewicht entspricht, zur Behandlung von chronischer oder akuter Niereninsuffizienz oder zur Wundheilung.

Das Gefäßendothel ist eine Schicht von Zellen, die die Blutgefäße auskleidet. Das Endothel trennt das Blut von anderen Gefäßschichten, wobei das Endothel nicht allein eine passive Barriere darstellt, sondern aktiv in die Regulation des Gefäßtonus eingreift. Dementsprechend wird auch von einer Endothel-abhängigen Vasodilatation gesprochen. Auf Grund seiner Lage ist das Endothel permanent dem hämodynamischen Stress und dem metabolischen Stress ausgesetzt. Bei pathogenen Zuständen, beispielsweise erhöhtem Blutdruck, erhöhten LDL-Werten, eingeschränkter Nierenfunktion oder erhöhtem Blutzucker, kommt es daher häufig zu funktionellen Defekten des Endothels, denen dann morphologisch fassbare Schäden, wie die Bildung atherosklerotischer Plaques folgen können. Ein sehr frühes Zeichen einer geänderten beziehungsweise reduzierten endothelialen Funktion beziehungsweise endothelialen Dysfunktion ist eine Verminderung der Endothel-abhängigen Vasodilatation.

Bei koronarer Herzkrankheit (KHK), aber auch bei vorhandenen Risikofaktoren ohne KHK, beispielsweise Hypertonie, Hyperlipoproteinämie oder Diabetes, äußern sich Endothel-Funktionsdefekte in einer verminderten Produktion von NO (=EDRF) und erhöhter Endothelin-Produktion. Hohe Plasmaspiegel von Endothelin führen zu abnormalem Zusammenwachsen von Zellen, Entzündungen, Gefäßwucherungen und starken Gefäßverengungen. Endothel-Funktionsstörungen sind zudem durch eine verstärkte Produktion von Adhäsionsmolekülen wie ICAM-1 und VCAM-1 gekennzeichnet, wodurch Thrombozyten und Monozyten in erhöhtem Maße am Endothel haften. Dadurch bedingt kommt es zu einer Erhöhung des Gefäßtonus. Somit bildet sich bei verschiedensten Systemen ein Ungleichgewicht heraus, wobei Vasokonstriktion, Adhäsion, Aggregation, Koagulation, Atherosklerose und Atherothrombose begünstigt werden. Selbst mentaler Stress führt zu einer messbaren Endothel-Dysfunktion, die bis zu 4 Stunden anhalten kann.

Endothelzellen sind auch an der Bildung neuer Blutgefäße beteiligt. Die Bildung von Blutgefäßen ist bei einer Vielzahl von Vorgängen, wie zum Beispiel der Embryogenese, dem weiblichen Reproduktionszyklus, der Wundheilung, dem Tumorwachstum und der Neovaskularisierung ischämischer Bereiche, von Bedeutung. Ursprünglich wurde die postnatale Blutgefäßbildung, also die Blutgefäßbildung nach der Geburt, hauptsächlich auf angiogenetische Prozesse zurückgeführt. Unter Angiogenese wird die Ausbildung neuer Blutgefäße durch das Aussprossen von Kapillaren aus einem bereits bestehenden Gefäßsystem verstanden. Bei der Angiogenese wird zunächst die die Blutgefäße umgebende Basalmembran mittels proteolytischer Enzyme abgebaut und die extrazelluläre Matrix im perivaskulären Raum fragmentiert. Die dabei freigesetzten angiogenen Stimuli bewirken, dass bereits vorhandene ausdifferenzierte Endothelzellen in Richtung des chemotaktischen Reizes migrieren, wobei sie gleichzeitig proliferieren und umgebaut werden. Durch die Aneinanderlagerung von Endothelzellen werden dann neue Gefäßschleifen mit einem kapillarförmigen Lumen ausgebildet. Danach setzt die Synthese einer neuen Basalmembran ein.

Neuere Untersuchungen zeigen jedoch, dass die Bildung neuer Blutgefäße im adulten Organismus nicht nur auf Angiogenese beruht, sondern auch auf vaskulogenetischen Mechanismen. Unter Vaskulogenese wird die Gefäßneubildung aus sich in situ differenzierenden endothelialen Vorläuferzellen verstanden. Das Dogma, dass die Vaskulogenese auf die Embryogenese beschränkt ist, wurde durch den Nachweis endothelialer Vorläuferzellen (EPC) im peripheren Blut von gesunden Menschen und Tieren widerlegt. Unter Verwendung von Tiermodellen konnte belegt werden, dass die aus dem Knochenmark stammenden endothelialen Vorläuferzellen aktiv an der Neovaskularisation beteiligt sind. Auch wurde gezeigt, dass sich in ischämischen Regionen eine spezifische CD34-positive Untergruppe von Leukozyten festsetzt, die Endothel-spezifische Antigene exprimiert. Darüber hinaus lassen sich aus CD133⁺- und CD34⁺-Zellen in vitro endotheliale Vorläuferzellen (EPC) gewinnen, die einen signifikanten Beitrag zur Blutgefäßbildung im adulten Organismus leisten (Asahara et al., Science, 275 (1997), 964-967; Crosby et al., Circ. Res., 87 (2000), 728-730; Gehling et al., Blood, 95 (2000), 3106-3112). Ferner wurde gezeigt, dass die Injektion von isolierten CD34⁺-Zellen oder kultivierten endothelialen Vorläuferzellen die Wiederherstellung des Blutflusses bei diabetischen Mäusen beschleunigt (Schatteman et al., J. Clin. Invest., 106 (2000), 571-578) und die Neovaskularisierung in vivo verbessert (Asahara et al., Circ. Res., 85 (1999), 221-228; Crosby et al., Circ. Res., 87 (2000), 728-730; Murohara et al., J. Clin. Invest., 105 (2000), 1527-1536). Darüber hinaus konnte gezeigt werden, dass eine durch CD34⁺-Zellen induzierte Neovaskularisierung die Herzfunktion verbessert (Kocher et al., Nat. Med., 7 (2001), 430-436).

Die der Mobilisierung und Differenzierung endothelialer Vorläuferzellen zugrunde liegenden Mechanismen sind jedoch noch nicht vollständig geklärt. Molekular- und zellbiologische Untersuchungen deuten darauf hin, dass verschiedene Cytokine und angiogene Wachstumsfaktoren stimulierend auf die Mobilisierung endothelialer Vorläuferzellen im Knochenmark wirken. So ist bekannt, dass proangiogene Faktoren wie VEGF und GM-CSF die Anzahl endothelialer Vorläuferzellen erhöhen können (Asahara et al., EMBO J., 18 (1999), 3964-3972; Takahashi et al., Nat. Med., 5 (1999), 434-438). Bei VEGF (Vascular Endothelial Growth Factor) handelt es sich um ein in verschiedenen Isoformen vorkommendes Protein, das an die beiden Tyrosinkinase-Rezeptoren VEGF-R1 (flt-1) und VEGF-R2 (flk-1) bindet, die beispielsweise auf der Oberfläche wachsender Endothelzellen vorkommen (Wernert et al., Angew. Chemie, 21 (1999), 3432-3435). Die Aktivierung der VEGF-Rezeptoren führt über den Ras-Raf-MAP-Kinase-Weg zur Expression von Proteinasen und spezifischen Integrinen auf der Oberfläche von Endothelzellen beziehungsweise endothelialen Vorläuferzellen und schließlich zur Initiierung der Proliferation und Migration dieser Zellen in Richtung des angiogenen Stimulus. Bei GM-CSF (Granulozyten-Makrophagen-Koloniestimulierender Faktor) handelt es sich um ein Cytokin, das bislang vor allem für die Stimulation weißer Blutkörperchen einschließlich Neutrophiler, Makrophagen und Eosinophiler bekannt war. Von PIGF (Plazenta-Wachstumsfaktor) ist bekannt, dass er die Mobilisierung von Endothel-Vorläuferzellen stimuliert, nicht jedoch deren Proliferation. Aus Untersuchungen von Llevadot et al. (J. Clin. Invest., 108 (2001), 399-405) geht hervor, dass HMG-CoA-Reduktase-Inhibitoren, insbesondere Statine, die als Lipid-senkende Medikamente eingesetzt werden und die Morbidität und Mortalität einer Koronarkrankheit reduzieren, endotheliale Vorläuferzellen mobilisieren können. Dimmeler et al. (J. Clin. Invest., 108 (2001), 391-397) konnten ferner zeigen, dass Statine wie Atorvastatin und Simvastatin die Differenzierung endothelialer Vorläuferzellen in mononukleären Zellen und CD34⁺-Stammzellen, die aus peripherem Blut isoliert wurden, in vitro und in vivo signifikant verbessern. So führte die Behandlung von Mäusen mit Statinen zu einer erhöhten Anzahl differenzierter endothelialer Vorläuferzellen, wobei Statine eine gleich starke Wirkung wie VEGF zeigten.

Die Stimulierung der Mobilisierung und/oder Differenzierung endothelialer Vorläuferzellen stellt eine wichtige neue therapeutische Strategie zur Erhöhung der postnatalen Neovaskularisation, insbesondere der Vaskulogenese, und zur Behandlung von Krankheiten, die im Zusammenhang mit einer Dysfunktion von endothelialen Vorläuferzellen und/oder Endothelzellen stehen, dar.

WO 02/14356 A offenbart die Verwendung von EPO zur Behandlung renaler Anämien aufgrund chronischer Niereninsuffizienz, nicht jedoch die Regeneration von Nierengewebe. Arcasoy Murat et al., Database Biosis, XP 002275417 (16.11.2001) offenbart die angiogenetische Funktion von EPO bei der Wundheilung.

Der vorliegenden Erfindung liegt das technische Problem zugrunde, eine Lehre bereitzustellen, gemäß der eine verbesserte Behandlung von mit einer Dysfunktion endothelialer Vorläuferzellen in Zusammenhang stehenden Erkrankungen, nämlich akuter und chronischer Niereninsuffizienz und Wunden, erreicht werden kann.

Die vorliegende Erfindung löst dieses technische Problem durch die Lehre, Erythropoietin gemäß der unabhängigen Patentansprüche einzusetzen.

Erfindungsgemäß wurde überraschenderweise festgestellt, dass eine Behandlung mit Erythropoietin zur physiologischen Mobilisierung endothelialer Vorläuferzellen führt, wobei die Anzahl zirkulierender endothelialer Vorläuferzellen erhöht und deren Differenzierung induziert wird, insbesondere in vergleichsweise niedrigen EPO-Dosen. Darüber hinaus werden unter bestimmten pathologischen Zuständen auftretende funktionelle Defizite der endothelialen Vorläuferzellen ausgeglichen. Erfindungsgemäß konnte gezeigt werden, dass bei Patienten mit chronischer Nierenerkrankung im Endstadium die Anzahl zirkulierender Stammzellen genauso hoch ist wie bei gesunden Probanden, diese jedoch bei diesen Patienten die Fähigkeit zur Differenzierung zu Endothelzellen über endotheliale Vorläuferzellen verloren haben. So ist bei Patienten mit chronischer Nierenerkrankung die Anzahl von Zellen, die adhäsionsfähig sind und einen endothelialen Zell-Phänotyp zeigen, gegenüber gesunden Probanden deutlich vermindert. Erfindungsgemäß wurde nun festgestellt, dass nach Behandlung dieser Patienten mit Erythropoietin die Anzahl zirkulierender Stammzellen signifikant um mehr als 50% ansteigt. Dabei erhöht sich insbesondere die Anzahl von Zellen, die einen endothelialen Phänotyp entwickeln, dramatisch. Wie mittels eines funktionellen Zellkulturtestes nachgewiesen, erhöht sich durch die Erythropoietin-Behandlung die bei den Patienten mit chronischer Nierenerkrankung beeinträchtigte Fähigkeit der endothelialen Vorläuferzellerzellen zur Adhäsion um das Dreifache. Die Fähigkeit von sich differenzierenden endothelialen Vorläuferzellen beziehungsweise von Endothelzellen zur Adhäsion ist eine der Grundvoraussetzungen zur Ausbildung neuer Gewebe und/oder Gefäße. Erythropoietin kann auf diese Weise die Neovaskularisierung, insbesondere die Vaskulogenese, in Geweben oder Organen, in denen entsprechende vaskulogene oder angiogene Stimuli freigesetzt werden, induzieren.

Erythropoietin (im Folgenden EPO genannt) kann zur Stimulierung der physiologischen Mobilisierung endothelialer Vorläuferzellen, der Proliferation endothelialer Vorläuferzellen, der Differenzierung der endothelialen Vorläuferzellen zu Endothelzellen und/oder zur Migration endothelialer Vorläuferzellen in Richtung eines vaskulogenen oder angiogenen Stimulus in einem menschlichen oder tierischen Körper, insbesondere einem adulten Organismus, verwendet werden. Erfindungsgemäß kann Erythropoietin daher in vorteilhafter Weise zur Stimulation der Gefäßneubildung durch Vaskulogenese in Geweben oder Organen eingesetzt werden, in denen pathologische Gefäßveränderungen vorliegen. Aufgrund der Stimulation endothelialer Vorläuferzellen durch Erythropoietin kann darüber hinaus auch die Bildung von Endothelgewebe induziert werden. Erfindungsgemäß kann Erythropoietin daher auch zur Behandlung von Krankheiten des menschlichen oder tierischen Körpers eingesetzt werden, die im Zusammenhang mit einer Dysfunktion von endothelialen Vorläuferzellen und/oder Endothelzellen im Zusammenhang stehen, nämlich chronischer oder akuter Niereninsuffizienz oder Wunden.

Im Zusammenhang mit der vorliegenden Erfindung wird unter "Erythropoietin" beziehungsweise "EPO" ein Stoff verstanden, der das Wachstum, die Differenzierung und die Reifung von Stammzellen über E-rythroblasten zu Erythrocyten steuert. Erythropoietin ist ein Glycoprotein, welches 166 Aminosäuren, drei Glycosylierungsstellen und ein Molekulargewicht von etwa 34.000 Da aufweist. Im Rahmen einer EPO-induzierten Differenzierung von Erythrocyten-Vorläuferzellen wird die Globinsynthese induziert und die Synthese des Häm-Komplexes sowie die Anzahl der Ferritin-Rezeptoren gesteigert. Dadurch kann die Zelle mehr Eisen aufnehmen und funktionelles Hämoglobin synthetisieren. In den reifen Erythrocyten bindet Hämoglobin den Sauerstoff. Somit nehmen die Erythrocyten beziehungsweise das in ihnen enthaltene Hämoglobin eine Schlüsselrolle für die Sauerstoffversorgung des Organismus ein. Ausgelöst werden diese Vorgänge durch die Wechselwirkung von EPO mit einem entsprechenden Rezeptor auf der Zelloberfläche der Erythrocyten-Vorläuferzellen (Graber und Krantz, Ann. Rev. Med. 29 (1978), 51-56).

Erythropoietin wird hauptsächlich in der Niere gebildet, in geringeren Anteilen jedoch auch in der Leber und im Hirn. Erythropoietin findet sich auch in geringen Mengen im Serum und wird unter physiologischen Bedingungen zumindest teilweise im Urin ausgeschieden. Patienten mit Niereninsuffizienz können nur unzureichend Erythropoietin (im Folgenden EPO genannt) bilden und leiden demzufolge an einer Anämie. Bekannt ist es, die Erythropoietin-Unterversorgung durch Verabreichung von Erythropoietin zu kompensieren. Weitere klinische Anwendungen von Erythropoietin liegen in der Applikation von Erythropoietin bei iatrogener Anämie nach Chemotherapie oder Strahlentherapie maligner Erkrankungen oder Virusinfektionen (EP 0 456 153 B1). Aus der US 4,732,889 ist es bekannt, Erythropoietin enthaltende Zusammensetzungen zur Behandlung von mit rheumatoider Arthritis assoziierter Anämie einzusetzen. Aus der WO 88/03808 ist die Behandlung von Hämochromatosis mittels EPO-haltiger Zusammensetzungen bekannt.

Der hier verwendete Begriff "Erythropoietin" umfasst EPO jeglicher Herkunft, insbesondere menschliches oder tierisches EPO. Der hier verwendete Begriff erfasst nicht nur die natürlich vorkommenden, das heißt Wildtyp-Formen von EPO, sondern auch seine Derivate, Analoga, Modifikationen, Muteine, Mutanten oder Sonstiges, solange sie die biologischen Wirkungen von Wildtyp-Erythropoietin zeigen.

Im Zusammenhang mit der vorliegenden Erfindung werden unter "Derivaten" funktionelle Äquivalente oder Abkömmlinge von Erythropoietin verstanden, die unter Beibehaltung der Erythropoietin-Grundstruktur durch Substitution von einem oder mehreren Atomen oder Molekülgruppen beziehungsweise -resten, insbesondere durch Substitution von Zuckerketten wie Ethylenglycol, erhalten werden und/oder deren Aminosäuresequenzen sich von der des natürlicherweise vorkommenden menschlichen oder tierischen Erythropoietin-Proteins an mindestens einer Position unterscheiden, die aber im wesentlichen einen hohen Grad an Homologie auf der Aminosäureebene und vergleichbare biologische Aktivität aufweisen. Derivate des Erythropoietins, wie sie beispielsweise in der vorliegenden Erfindung eingesetzt werden können, sind unter anderem bekannt aus der WO 94/25055, der EP 0 148 605 B1 oder der WO 95/05465.

"Homologie" bedeutet insbesondere eine Sequenzidentität von mindestens 80 %, vorzugsweise mindestens 85 % und besonders bevorzugt mindestens mehr als 90 %, 95 %, 97 % und 99 %. Der dem Fachmann bekannte Ausdruck der "Homologie" bezeichnet somit den Grad der Verwandtschaft zwischen zwei oder mehreren Polypeptid-Molekülen, der durch die Übereinstimmung zwischen den Sequenzen bestimmt wird. Dabei kann eine Übereinstimmung sowohl eine identische Übereinstimmung als auch ein konservativer Aminosäureaustausch bedeuten.

Erfindungsgemäß umfasst der Begriff "Derivat" auch Fusionsproteine, bei denen am N-terminalen Teil beziehungsweise am C-terminalen Teil funktionelle Domänen eines anderen Proteins vorhanden sind. In einer Ausführungsform der Erfindung kann es sich bei diesem anderen Protein beispielsweise um GM-CSF, VEGF, PIGF, ein Statin oder einen anderen Faktor handeln, der eine stimulierende Wirkung auf endotheliale Vorläuferzellen aufweist. In einer weiteren Ausführungsform der Erfindung kann es sich bei dem anderen Protein auch um einen Faktor handeln, der stimulierende Wirkung auf ausdifferenzierte Endothelzellen hat, beispielsweise um Angiogenin oder bFGF (basic fibroblast growth factor). Von bFGF ist bekannt, dass dieser Wachstumsfaktor eine starke mitogene und chemotaktische Aktivität auf Endothelzellen ausübt.

Die Unterschiede zwischen einem Erythropoietin-Derivat und nativem Erythropoietin können beispielsweise durch Mutationen, wie zum Beispiel Deletionen, Substitutionen, Insertionen, Anlagerungen, Basenaustausche und/oder Rekombinationen der die Erythropoietin-Aminosäuresequenzen codierenden Nucleotidsequenzen entstanden sein. Selbstverständlich kann es sich dabei auch um natürlicherweise auftretende Sequenzvariationen handeln, beispielsweise um Sequenzen aus einem anderen Organismus oder um Sequenzen, die auf natürliche Weise mutiert wurden, oder Mutationen, die mit Hilfe üblicher, auf dem Fachgebiet bekannter Mittel, beispielsweise chemische Agenzien und/oder physikalische Agenzien, gezielt in die Erythropoietin codierenden Nucleinsäuresequenzen eingeführt wurden. Im Zusammenhang mit der Erfindung umfasst der Begriff "Derivat" daher auch mutierte Erythropoietin-Moleküle, also Erythropoietin-Muteine.

Erfindungsgemäß können auch Peptid- oder Protein-Analoga von Erythropoietin eingesetzt werden. Im Zusammenhang mit der vorliegenden Erfindung umfasst der Begriff "Analoga" Verbindungen, die keine mit der Erythropoietin-Aminosäuresequenz identische Aminosäuresequenz aufweisen, deren dreidimensionale Struktur jedoch der von Erythropoietin stark ähnelt und die daher eine vergleichbare biologische Aktivität aufweisen. Bei Erythropoietin-Analoga kann es sich beispielsweise um Verbindungen handeln, die die für die Bindung von Erythropoietin an dessen Rezeptoren verantwortlichen Aminosäurereste in geeigneter Konformation enthalten und die daher die essentiellen Oberflächeneigenschaften des Erythropoietin-Bindungsbereiches nachahmen können. Derartige Verbindungen sind beispielsweise in Wrighton et al., Science, 273 (1996), 458, beschrieben.

Das erfindungsgemäß eingesetzte EPO kann auf verschiedene Weise hergestellt werden, beispielsweise durch Isolierung aus menschlichem Urin oder aus dem Urin oder Plasma (einschließlich Serum) von an aplastischer Anämie leidenden Patienten (Miyake et al., J.B.C. 252 (1977), 5558). Menschliches EPO kann beispielsweise auch aus Gewebekulturen von menschlichen Nierenkrebszellen (JA-OS 55790/1979), aus menschlichen Lymphoblastenzellen, die die Fähigkeit zur Bildung von menschlichem EPO aufweisen (JA-OS 40411/1982) und aus einer durch Zellfusion einer menschliche Zelllinien erhaltenen Hybridomakultur gewonnen werden. EPO kann auch durch gentechnologische Verfahren hergestellt werden, indem man mittels geeigneter DNA oder RNA, die für die entsprechende Aminosäuresequenz des EPO codiert, gentechnisch das erwünschte Protein herstellt, zum Beispiel in einem Bakterium, einer Hefe, einer Pflanzen- oder Tierzelllinie. Derartige Verfahren sind beispielsweise in der EP 0 148 605 B2 oder der EP 0 205 564 B2 sowie der EP 0 411 678 B1 beschrieben.

Im Zusammenhang mit der vorliegenden Erfindung werden unter "endothelialen Vorläuferzellen" (endotheliale Progenitorzellen; EPC) im Blutkreislauf zirkulierende Zellen verstanden, die die Fähigkeit zur Differenzierung zu Endothelzellen besitzen. Bei den während der Embryonalentwicklung vorkommenden endothelialen Vorläuferzellen handelt es sich um Angioblasten. Bei den im adulten Organismus vorkommenden endothelialen Vorläuferzellen handelt es sich um Angioblasten-ähnliche Zellen, die aus mononucleären Zellen, insbesondere CD34⁻CD14⁺-Monozyten, und/oder CD34⁺-Stammzellen, die aus peripherem Blut isoliert wurden, gewonnen werden können.

Im Zusammenhang mit der vorliegenden Erfindung wird unter "Mobilisierung" oder "physiologischer Mobilisierung" der Prozess des Aktivierens von Stammzellen und/oder Progenitorzellen aus dem Knochenmark durch Wachstumsfaktoren verstanden, wobei die Stammzellen beziehungsweise Progenitorzellen in den Blutkreislauf, insbesondere ins periphere Blut, gelangen.

Im Zusammenhang mit der vorliegenden Erfindung wird unter "Proliferation" die Fähigkeit von Zellen zur Vergrößerung und zur anschließenden Teilung in zwei oder mehr Tochterzellen verstanden. Die EPOvermittelte Stimulierung endothelialer Vorläuferzellen betrifft somit insbesondere die Anzahl und damit das Teilungsverhalten endothelialer Vorläuferzellen.

Im Zusammenhang mit der vorliegenden Erfindung wird unter "Differenzierung" endothelialer Vorläuferzellen die Entwicklung von aus dem Knochenmark stammenden mononukleären Zellen über endotheliale Vorläuferzellen zu Endothelzellen verstanden. Unter "Endothelzellen" werden die Zellen verstanden, die das Endothel, das heißt die einschichtige zellige Auskleidung von Gefäßen und serösen Höhlen, bilden. Endothelzellen sind dadurch charakterisiert, dass sie gefäßaktive Substanzen, beispielsweise gefäßerweiternde Substanzen wie EDRF (endothelial derived relaxing factor) oder konstringierenden Substanzen wie Endothelin, Faktoren zur Hemmung oder Aktivierung der Blutgerinnung und Faktoren zur Regulation der Gefäßpermeabilität freisetzen. Endothelzellen synthetisieren auch Komponenten des subendothelialen Bindegewebes, insbesondere Collagene vom Typ IV und V, Zellhaftproteine wie Laminin, Fibronektin und Thrombospondin, Wachstumsfaktoren, beispielsweise für glatte Muskelzellen, und Faktoren zur Gefäßneubildung.

Im Zusammenhang mit der vorliegenden Erfindung wird unter "Migration" der endothelialen Vorläuferzellen verstanden, dass die im Blutkreislauf befindlichen endothelialen Vorläuferzellen in Richtung eines vaskulogenen oder angiogenen Stimulus wandern und sich im Bereich des vaskulogenen oder angiogenen Stimulus konzentrieren. Unter einem "vaskulogenen Stimulus" wird ein chemischer Reiz in einem Gewebe oder Blutgefäß eines menschlichen oder tierischen Körpers verstanden, der spezifisch auf endotheliale Vorläuferzellen wirkt und deren Wanderung zu der Stelle des Körpers bewirkt, von der der chemische Reiz ausgeht. Durch den vaskulogenen Stimulus wird auf diese Weise der Prozess der Vaskulogenese induziert. Unter einem "angiogenen Stimulus" wird ein chemischer Reiz in einem Gewebe oder Blutgefäß eines menschlichen oder tierischen Körpers verstanden, der spezifisch auf ausdifferenzierte Endothelzellen wirkt und deren Migration zu der Stelle des Körpers bewirkt, von der der chemische Reiz ausgeht. Der angiogene Stimulus bewirkt auf diese Weise eine Induzierung von Angiogenese.

In der Erfindung ist die Verwendung von Erythropoietin und/oder Derivaten davon zur Herstellung einer pharmazeutischen Zusammensetzung zur Therapie von Krankheitszuständen oder Krankheiten des menschlichen oder tierischen Körpers, die im Zusammenhang mit einer Dysfunktion von endothelialen Vorläuferzellen stehen, oder von Folgerkrankungen davon vorgesehen, nämlich akuter oder chronischer Niereninsuffizienz oder Wunden.

Im Zusammenhang mit der vorliegenden Erfindung werden unter "Krankheiten", "Krankheitszuständen" oder "Erkrankungen" Störungen der Lebensvorgänge in Organen oder im gesamten Organismus mit der Folge von subjektiv empfundenen beziehungsweise objektiv feststellbaren körperlichen, seelischen beziehungsweise geistigen Veränderungen verstanden. Erfindungsgemäß handelt es sich insbesondere um Krankheiten, die mit einer Dysfunktion von endothelialen Vorläuferzellen im Zusammenhang stehen, das heißt Krankheiten, die entweder die Folge einer derartigen Dysfunktion dieser Zellen sind oder die durch diese Zellen vermittelt werden. Unter "Folgerkrankungen" werden Sekundärkrankheiten verstanden, das heißt eine zu einem primären Krankheitsbild hinzutretende zweite Erkrankung.

Im Zusammenhang mit der vorliegenden Erfindung wird unter einer "Dysfunktion" von endothelialen Vorläuferzellen eine Störung wesentlicher Zellfunktionen wie Stoffwechselleistungen, Reizbeantwortung, Motilität, Teilungsverhalten oder Differenzierungsverhalten dieser Zellen verstanden. Eine Dysfunktion von endothelialen Vorläuferzellen kann beispielsweise darin bestehen, dass diese Zellen nicht oder nur in ungenügendem Maße proliferieren. Da durch die Verwendung von Erythropoietin die Proliferation endothelialer Vorläuferzellen stimuliert wird, kann dadurch das defizitäre Teilungsverhalten sowohl von endothelialen Vorläuferzellen als auch von bereits ausdifferenzierten Endothelzellen ausgeglichen und die Anzahl von endothelialer Vorläuferzellen beziehungsweise Endothelzellen erhöht werden. Eine Dysfunktion endothelialer Vorläuferzellen kann beispielsweise in der gestörten Fähigkeit dieser Zellen zur Differenzierung zu Endothelzellen bestehen. Die Dysfunktion von endothelialen Vorläuferzellen kann auch bedingt sein durch deren gestörte Fähigkeit zur Adhäsion und/oder deren gestörte Fähigkeit zur Migration in Richtung eines angiogenen oder vaskulogenen Stimulus. Solche Dysfunktionen von endothelialen Vorläuferzellen können dazu führen, dass beispielsweise die Neubildung von Endothelgewebe und/oder Vaskulogenese beeinträchtigt oder verhindert wird. Eine Dysfunktion von endothelialen Vorläuferzellen kann auch pathogen bedingt sein, beispielsweise durch Hypertonie, Hyperlipoproteinämie, Urämie oder Diabetes. Die Dysfunktion von endothelialen Vorläuferzellen kann sich beispielsweise in einer verminderten Produktion von NO (=EDRF) durch NO-Synthasen (NOS) aus L-Arginin, erhöhter Endothelin-Produktion und/oder in einer verstärkten Produktion von Adhäsionsmolekülen wie ICAM-1 und VCAM-1 äußern.

Erfindungsgemäß handelt es sich bei den Krankheiten, die mit einer Dysfunktion von endothelialen Vorläuferzellen im Zusammenhang stehen um chronische oder akute Niereninsuffizienz, insbesondere terminale Niereninsuffizienz oder Wundheilung sowie Folgeerkrankungen davon.

Unter "Niereninsuffizienz" wird im Zusammenhang mit der vorliegenden Erfindung die eingeschränkte Fähigkeit der Nieren zur Ausscheidung harnpflichtiger Substanzen verstanden, wobei in fortgeschrittenen Stadien auch die Regulationsfähigkeit des Elektrolyt-, Wasser- und Säure-Basen-Haushalts verloren geht. Die terminale Niereninsuffizienz ist durch einen Zusammenbruch der exkretorischen und endokrinen Nierenfunktion gekennzeichnet.

Bei der Niereninsuffizienz kann es sich erfindungsgemäß um akute Niereninsuffizienz handeln, die auch als akutes Nierenversagen, Schockniere oder Schockanurie bezeichnet wird. Akute Niereninsuffizienz ist durch einen plötzlichen partiellen oder totalen Verlust der exkretorischen Nierenfunktion als Folge eines meist reversiblen Nierenschadens gekennzeichnet. Ursache können eine Minderperfusion durch Hypovolämie, Hypotonie und Dehydratation in Folge von Blutverlusten (Polytrauma, gastrointestinale oder postpartale Blutung, große operative Eingriffe an Herz, Gefäßen, Abdomen oder Prostata), Schock (Myokardinfarkt, Embolie), schwerer Infektionen (Sepsis, Peritonitis, Cholezystitis), Hämolyse (hämolytisch-urämisches Syndrom, paroxysmale Hämoglobinurie, Transfusionszwischenfall), Myolyse (Crush-Syndrom, Rhabdomyolyse, Myositis, Verbrennungen), Wasser- und Elektrolytverlusten (massives Erbrechen, Durchfälle, exzessives Schwitzen, Ileus, akute Pankreatidis). Weitere Ursachen können Nephrotoxine wie exogene Toxine, beispielsweise Anilin, Glykolverbindungen, Methanol und ähnliche, oder endogene Toxine, beispielsweise Myoglobin und Oxalate, sein. Weitere Ursachen für akute Niereninsuffizienz sind Nierenkrankheiten, beispielsweise entzündliche Nephropatien oder Abstoßungsreaktionen nach Nierentransplantation, sein. Akute Niereninsuffizienz kann auch durch eine Harnstauung in Folge von Harnabflussbehinderungen verursacht werden. Die erfindungsgemäße Behandlung akuter Niereninsuffizienz mit Erythropoietin führt erfindungsgemäß zur Verhinderung oder zumindest zur Verminderung der Progression der akuten Niereninsuffizienz.

Bei der Niereninsuffizienz kann es sich erfindungsgemäß auch um eine chronische Niereninsuffizienz handeln. Ursachen der chronischen Niereninsuffizienz sind vaskuläre, glomeruläre und tubulointerstitielle Nierenerkrankungen, Infektionen sowie angeborene oder erworbene Strukturdefekte. Ursachen der chronischen Niereninsuffizienz sind unter anderem chronische Glomerulopathie, chronische Pyelonephritis, Analgetika-Nephropathie, obstruktive U-ropathie sowie Arterio- und Arteriolosklerose. Die chronische Niereninsuffizienz geht terminal in die Urämie über. Die erfindungsgemäße Behandlung von chronischer Niereninsuffizienz mit Erythropoietin führt erfindungsgemäß zur Verminderung der Progression von chronischer Niereninsuffizienz.

Im Zusammenhang mit der vorliegenden Erfindung werden unter "Wundheilung" die physiologischen Vorgänge zur Regeneration zerstörten Gewebes beziehungsweise zum Verschluss einer Wunde, insbesondere die Neubildung von Bindegewebe und Kapillaren verstanden. Bei der Wundheilung kann es sich um eine primäre Wundheilung (Sanatio per primam intentionem) handeln, die durch einen raschen und komplikationslosen Verschluss und weitgehende Restitutio ad integrum infolge minimaler Bindegewebeneubildung zwischen den gut durchbluteten und gegebenenfalls adaptierten Wundrändern einer sauberen Wunde gekennzeichnet ist. Bei Wunden mit weiter auseinanderliegenden, insbesondere gequetschten oder nekrotischen Wundrändern beziehungsweise Wundinfektionen erfolgt eine verzögerte sekundäre Wundheilung (Sanatio per secundam intentionem), bei der es infolge einer (a)bakteriellen Entzündung zur Auffüllung des Gewebedefektes mit Granulationsgewebe und ausgedehnteren Bildung von Narbengewebe kommt. Die Epithelisierung vom Rand her bildet den Abschluss der Wundheilung. Die Wundheilung untergliedert sich in die drei Phasen, nämlich Latenzphase, Proliferationsphase und Reparationsphase. Die Latenzphase wiederum untergliedert sich in die exsudative Phase mit Schorfbildung, insbesondere in den ersten Stunden nach Entstehung der Wunde, und die resorptive Phase mit kataboler Autolyse, die sich über einen Zeitraum von ein bis drei Tagen nach Entstehung der Wunde erstreckt. Die Proliferationsphase ist durch eine anabole Reparation mit Bildung von Collagen durch Fibroblasten gekennzeichnet und tritt am vierten bis siebten Tag nach Entstehung der Wunde auf. Ab dem achten Tag nach Entstehung der Wunde tritt die Reparationsphase auf, die durch Umwandlung des Granulationsgewebes in eine Narbe gekennzeichnet ist.

Unter einer "Wunde" wird im Zusammenhang mit der vorliegenden Erfindung eine Unterbrechung des Zusammenhangs von Körpergeweben mit oder ohne Substanzverlust verstanden, die durch mechanische Verletzung oder physikalisch bedingte Zellschädigung verursacht wird. Wundformen sind mechanische Wunden, thermische Wunden, chemische Wunden, strahlenbedingte Wunden und krankheitsbedingte Wunden. Mechanische Wunden entstehen durch äußere Gewalteinwirkung und treten vor allem als Schnitt- und Stichwunden, Quetsch-, Platz-, Riss- und Schürfwunden, Kratz- und Bisswunden und Schusswunden auf. Thermische Wunden entstehen durch Einwirkung von Hitze oder Kälte. Chemische Wunden entstehen insbesondere durch Verätzung mit Säuren oder Laugen. Strahlenbedingte Wunden entstehen beispielsweise durch Einwirkung aktinischer und ionisierender Strahlung. Krankheitsbedingt auftretende Wunden sind insbesondere stauungsbedingte Wunden, traumatische Wunden, diabetische Wunden etc.. Erfindungsgemäß ist insbesondere vorgesehen, dass zur Wundheilung Erythropoietin vorzugsweise topisch oder intravenös appliziert wird.

Die vorliegende Erfindung betrifft daher die Verwendung von Erythropoietin zur Herstellung einer pharmazeutischen Zusammensetzung zur Therapie von chronischer oder akuter Niereninsuffizienz, insbesondere terminaler Niereninsuffizienz oder Wundheilung und Folgeerkrankungen davon.

Erfindungsgemäß ist vorgesehen, dass Erythropoietin in einer therapeutisch wirksamen Dosis an einen Patienten verabreicht wird, die ausreicht, den Zustand einer vorgenannten Krankheit, insbesondere einer Krankheit, die im Zusammenhang mit einer Dysfunktion von endothelialen Vorläuferzellen steht, zu heilen oder diesem Zustand vorzubeugen, die Progression einer solchen Krankheit zu stoppen und/oder die Symptome einer solchen Krankheit zu lindern. Die an einen Patienten zu verabreichende Dosis hängt von vielen Faktoren, beispielsweise dem Alter, Körpergewicht und Geschlecht des Patienten, der Schwere der Erkrankungen usw. ab.

Erfindungsgemäß wird Erythropoietin in allen Verwendungen, Verfahren und Zusammensetzungen der vorliegenden Lehre in sehr geringen Mengen, die unterhalb der bekanntermaßen eingesetzten Mengen liegen, verwendet, wobei insbesondere in vivo, d.h. pro Patient, EPO-Dosen von 200 bis 2000 Einheiten (IU; International Units)/Woche, vorzugsweise Dosen von 500 bis 2000 IU/Woche, je nach Schwere der Erkrankung und in Abhängigkeit von der Nierenfunktion, verabreicht werden. Bei den erfindungsgemäß vorgesehenen Dosen von 200 bis 2000 Einheiten (IU)/Woche und pro Patient, insbesondere von 500 bis 2000 IU/Woche und pro Patient, handelt es sich um subpolycythämische Dosen, das heißt Dosen, die nicht zu einer Erythrozythose mit Hämatokritwerten von mehr als 50% führen. Die erfindungsgemäß vorgesehenen subpolycythämischen Dosen entsprechen wöchentlichen Dosen von etwa 1 bis 90 Einheiten (IU) EPO/kg Körpergewicht, insbesondere 1 bis 45 IU EPO/kg Körpergewicht, oder einer vergleichbaren Wochendosis Aranesp von 0,005 bis 0,45 µg/kg Körpergewicht, insbesondere 0,005 bis 0,225 µg/kg Körpergewicht. Bei Aranesp handelt es sich um ein doppelt PEG-yliertes EPO. Die Dosis von 200 bis 2000 Einheiten/Woche pro Patient, insbesondere von 500 bis 2000 IU/Woche und pro Patient, die erfindungsgemäß zur Behandlung von Krankheiten oder Krankheitszuständen vorgesehen ist, die mit einer Dysfunktion endothelialer Vorläuferzellen einhergehen, ist im Vergleich zu der üblicherweise zur Therapie renaler Anämie eingesetzten Anfangsdosis von 50-150 IU/kg Körpergewicht/Woche (in der Regel beginnend mit 4000-8000 IU/Woche, bei nicht befriedigendem Therapieergebnis jedoch auch wesentlich höher) sehr niedrig.

Die Erfindung betrifft die Verwendung von Erythropoietin und/oder dessen Derivaten als Wirkstoff zur Herstellung einer pharmazeutischen Zusammensetzung oder eines Arzneimittels zur Therapie von den genannten Krankheitszuständen oder Krankheiten, die mit einer Dysfunktion von endothelialen Vorläuferzellen im Zusammenhang stehen.

Erfindungsgemäß wird unter einem "Wirkstoff" ein körpereigener oder körperfremder Stoff verstanden, der bei Kontakt mit Zielmolekülen oder Zielzellen oder Zielgeweben spezifische Funktionen von Geweben, Organen oder Organismen in differenzierter Weise beeinflusst. Erfindungsgemäß ist also vorgesehen, dass Erythropoietin als Wirkstoff der erfindungsgemäßen pharmazeutischen Zusammensetzung bei Kontakt mit endothelialen Vorläuferzellen deren Proliferations-, Differenzierungs- und/oder Migrationsverhalten in einem menschlichen oder tierischen Organismus so beeinflusst, dass Dysfunktionen von endothelialen Vorläuferzellen ausgeglichen und die infolge dieser Dysfunktionen auftretenden Krankheiten wirksam bekämpft, gelindert oder beseitigt werden können beziehungsweise diesen Krankheiten wirksam vorgebeugt werden kann.

Im Zusammenhang mit der vorliegenden Erfindung wird unter einer "pharmazeutischen Zusammensetzung" oder einem "Arzneimittel" ein zu diagnostischen, therapeutischen und/oder prophylaktischen Zwecken verwendetes, also ein die Gesundheit eines menschlichen oder tierischen Körpers förderndes oder wiederherstellendes Gemisch verstanden, das mindestens einen natürlichen oder synthetisch hergestellten Wirkstoff umfasst, der die therapeutische Wirkung hervorruft. Die pharmazeutische Zusammensetzung kann sowohl ein festes als auch ein flüssiges Gemisch sein. Beispielsweise kann eine den Wirkstoff umfassende pharmazeutische Zusammensetzung einen oder mehrere pharmazeutisch verträgliche Komponenten enthalten. Darüber hinaus kann die pharmazeutische Zusammensetzung üblicherweise auf dem Fachgebiet verwendete Zusatzstoffe, beispielsweise Stabilisatoren, Fertigungsmittel, Trennmittel, Sprengmittel, Emulgatoren oder andere üblicherweise zur Herstellung pharmazeutischer Zusammensetzung verwendete Stoffe umfassen.

Erfindungsgemäß ist insbesondere die Verwendung von Erythropoietin und/oder eines Derivats davon als Wirkstoff zur Herstellung eines Arzneimittels zur Therapie von akuter oder chronischer Niereninsuffizienz, insbesondere terminaler Niereninsuffizienz oder Wundheilung sowie Folgeerkrankungen davon vorgesehen.

Die erfindungsgemäße pharmazeutische Zusammensetzung kann sowohl zur topischen als auch zur systemischen Verabreichung geeignet sein.

In einer bevorzugten Ausführungsform der Erfindung ist vorgesehen, dass die pharmazeutische Zusammensetzung zur parenteralen, insbesondere intravenösen, intramuskulären, intrakutanen oder subkutanen Verabreichung verwendet wird. Vorzugsweise weist das Erythropoietin enthaltende Arzneimittel die Form einer Injektion oder Infusion auf.

In einer weiteren Verwendung ist vorgesehen, dass die Erythropoietin enthaltende pharmazeutische Zusammensetzung oral verabreicht wird. Beispielsweise wird das Erythropoietin enthaltende Arzneimittel in einer flüssigen Darreichungsform wie einer Lösung, Suspension oder Emulsion, oder einer festen Darreichungsform wie einer Tablette verabreicht.

In einer weiteren Verwendung ist vorgesehen, dass die pharmazeutische Zusammensetzung zur pulmonalen Verabreichung oder zur Inhalation geeignet ist. Erfindungsgemäß ist also vorgesehen, dass Erythropoietin in therapeutisch wirksamer Weise direkt an die Lungen des Patienten verabreicht wird. Diese Form der Verabreichung von Erythropoietin ermöglicht eine schnelle Abgabe einer Erythropoietin-Dosis an einen Patienten, ohne dass eine Injektion vorgenommen werden muss. Bei Aufnahme von Erythropoietin über die Lungen können erhebliche Erythropoietin-Mengen über die Lunge an den Blutkreislauf abgegeben werden, was im Blutkreislauf zu erhöhten Erythropoietin-Mengen führt. In einer bevorzugten Ausführungsform der Erfindung handelt es sich bei der über die Lunge aufzunehmenden pharmazeutischen Zusammensetzung um eine wässrige oder nichtwässrige Lösung oder um ein Trockenpulver. Bei Vorliegen des pulmonal zu verabreichende, Erythropoietin enthaltenden Arzneimittel in Form eines Trockenpulvers umfasst dieses vorzugsweise Erythropoietinenthaltende Partikel, wobei die Partikel einen Durchmesser von weniger als 10 µm aufweisen, so dass das Medikament auch distale Bereiche der Lunge des Patienten erreichen kann. In einer besonders bevorzugten Ausführungsform der Erfindung ist vorgesehen, dass das pulmonal zu verabreichende Medikament in Form eines Aerosols vorliegt.

Eine besonders bevorzugte Ausführungsform der Erfindung betrifft die Verwendung von Erythropoietin zur Herstellung einer pharmazeutischen Zusammensetzung zur Therapie von den genannten Krankheiten, die mit einer Dysfunktion von endothelialen Vorläuferzellen im Zusammenhang stehen, wobei die pharmazeutische Zusammensetzung neben Erythropoietin als Wirkstoff mindestens einen weiteren zusätzlichen Wirkstoff zur Stimulierung von endothelialen Vorläuferzellen enthält.

Vorzugsweise handelt es sich bei dem weiteren Wirkstoff um einen Wirkstoff, der insbesondere die physiologische Mobilisierung endothelialer Vorläuferzellen aus dem Knochenmark stimuliert. Erfindungsgemäß kann es sich bei dem weiteren Wirkstoff aber auch um einen Wirkstoff handeln, der insbesondere das Teilungsverhalten, also die Proliferation endothelialer Vorläuferzellen stimuliert. Erfindungsgemäß besteht jedoch auch die Möglichkeit, dass der weitere Wirkstoff insbesondere das Differenzierungsverhalten und/oder das Migrationsverhalten endothelialer Vorläuferzellen stimuliert. Besonders bevorzugt handelt es sich bei dem weiteren, endotheliale Vorläuferzellen stimulierenden Wirkstoff um VEGF, PIGF, GM-CSF, einen HMG-CoA-Reduktase-Inhibitor, insbesondere ein Statin wie Simvastatin, Mevastatin oder Atorvastatin, und/oder einen NO-Donator, insbesondere L-Arginin.

Erfindungsgemäß ist auch vorgesehen, dass der mindestens eine weitere Wirkstoff insbesondere ausdifferenzierte Endothelzellen, das heißt deren Proliferation und/oder Migration stimuliert, nicht jedoch endotheliale Vorläuferzellen. Besonders bevorzugt handelt es sich dabei um bFGF (basic fibroblast growth factor) oder Angiogenin.

Weitere vorteilhafte Ausgestaltungen der Erfindung ergeben sich aus den Unteransprüchen.

Die Erfindung wird anhand der folgenden Figuren und Beispiele näher erläutert.

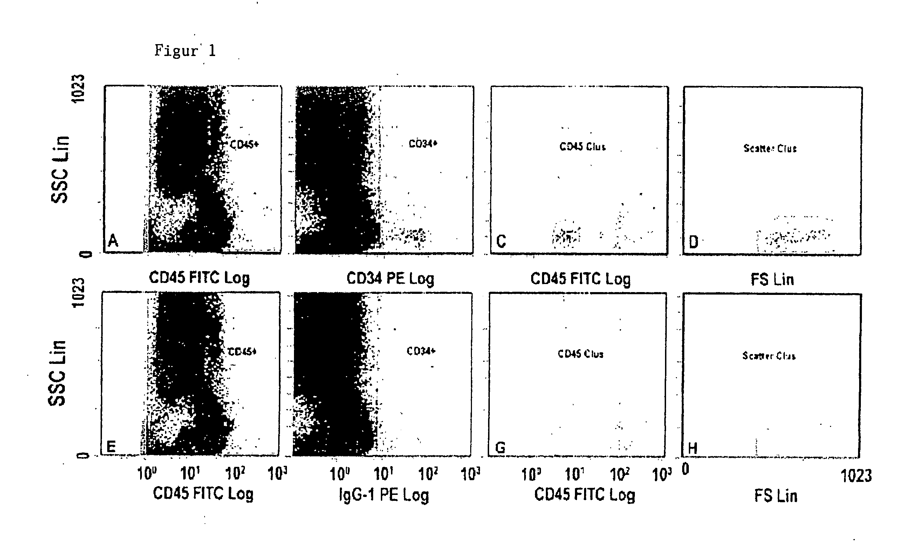

Figur 1 zeigt die Ergebnisse einer FACS-Analyse zirkulierender CD34⁺-Stammzellen (cSC). (A-D) : Patientenproben; (E-F): isotypische Kontrollen. cSC wurden anhand der zusätzlichen Expression des CD34-Markers (B und F), anhand der charakteristischen geringen bis mittleren CD45-Antigen-Expression (Cund G) und anhand der charakteristischen Lichtstreuungs-Eigenschaften (D und H) identifiziert. Die absolute cSC-Anzahl wurde pro 100.000 Mono-und Lymphozyten berechnet.

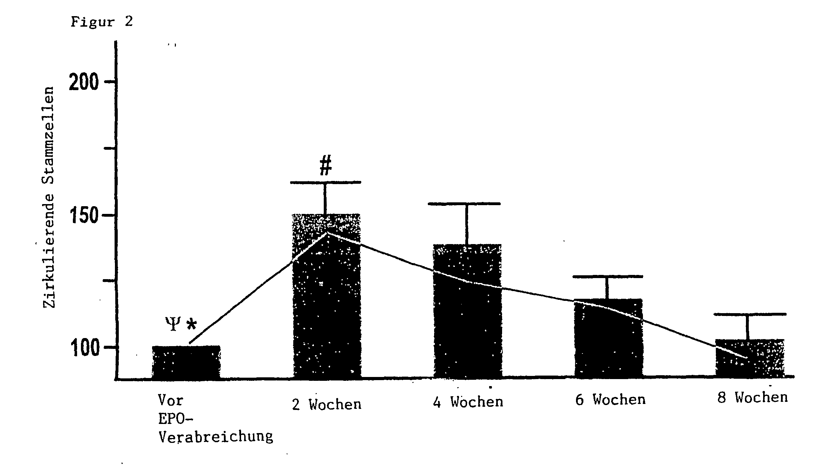

Figur 2 zeigt eine quantitative Bestimmung zirkulierender Stammzellen mittels Durchflusszytometrie. Die Figur zeigt die zeitabhängige Wirkung einer Erythropoietin-Behandlung unter Verwendung von rhEPO (rekombinantes menschliches Erythropoietin) nach 0, 2, 4, 6 und 8 Wochen. n=11, die Werte entsprechen Mittelwerten +/-Standardabweichung. Mediane als Linie dargestellt.
*: p < 0,01 im Vergleich zu 2 Wochen; □□□□p < 0,05 im Vergleich zu 4 Wochen, #: p < 0,05 im Vergleich zu 8 Wochen.

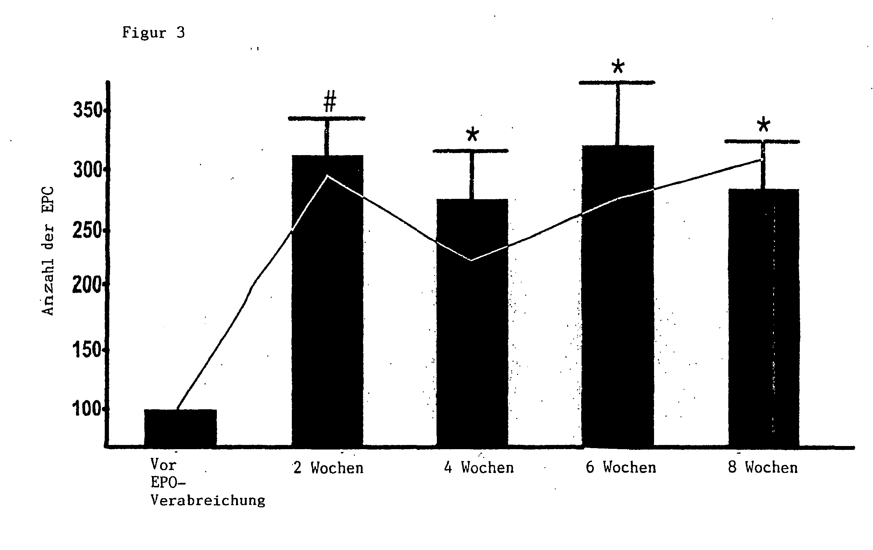

Figur 3 zeigt eine quantitative Bestimmung kultivierter endothelialer Vorläuferzellen (EPC). Die Figur zeigt, dass die rhEPO-Behandlung die relative Anzahl von EPCs erhöht. EPCs wurden vor der Behandlung renaler Patienten mit rhEPO sowie 2, 4, 6 und 8 Wochen nach Behandlung der Patienten mit rhEPO isoliert und anhand ihrer Adhäsionsfähigkeit und der beiden Marker acLDL-Dil und UEA-1 FITC charaketrisiert. n=11, die Werte entsprechen Mittelwerten +/-Standardabweichung. Mediane als Linie dargestellt.
*: p < 0,01 gegenüber dem Zeitraum vor Behandlung; #: p < 0,001 gegenüber dem Zeitraum vor Behandlung.

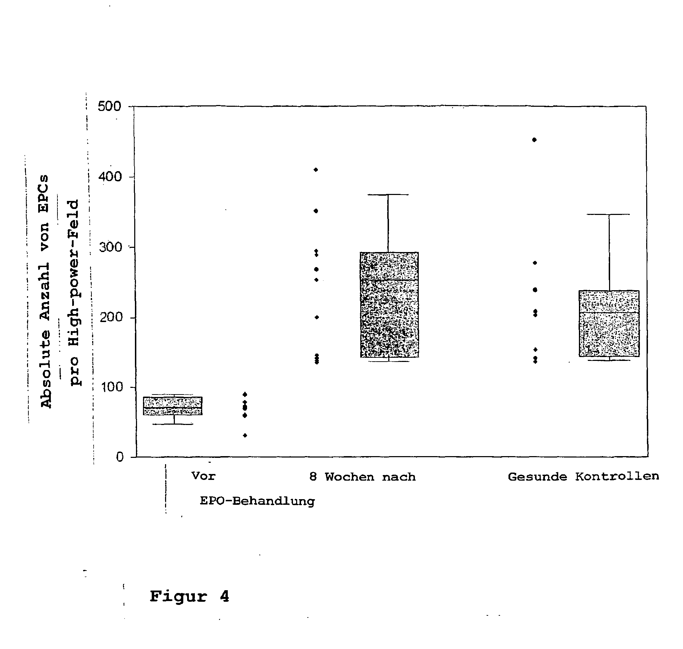

Figur 4 zeigt die quantitative Bestimmung kultivierter endothelialer Vorläuferzellen (EPC). Die Figur zeigt, dass die absolute Anzahl von EPCs vor Einsetzen einer rhEPO-Therapie signifikant gegenüber gesunden alters- und geschlechtsgematchten Probanden verringert ist. Patienten mit renaler Anämie zeigen also eine deutliche EPC-Dysfunkion gegenüber Kontrollpersonen. 8 Wochen nach Beginn der rhEPO-Therapie zur renalen Anämie wird diese verminderte Anzahl funktioneller EPC ausgeglichen.

EPCs wurden vor der Behandlung renaler Patienten mit rhEPO sowie 2, 4, 6 und 8 Wochen nach Behandlung der Patienten mit rhEPO isoliert und anhand ihrer Adhäsionsfähigkeit und der beiden Marker acLDL-Dil und UEA-1 FITC charaketrisiert. n=11. Dargestellt ist exemplarisch der 8-Wochenverlauf und die gesamten Kontrollen. Die absoluten Werte sind einerseits als Einzelwerte dargestellt. Zusätzlich sind Boxplots dargestellt (90-er/75-er/50-er/25-er und 10-er Percentile sowie der Mittelwert). Als gesunde Kontrolle dienten alters- und geschlechtsgematchte Probanden, bei denen analog EPCs isoliert und charakterisiert wurden (n =11).

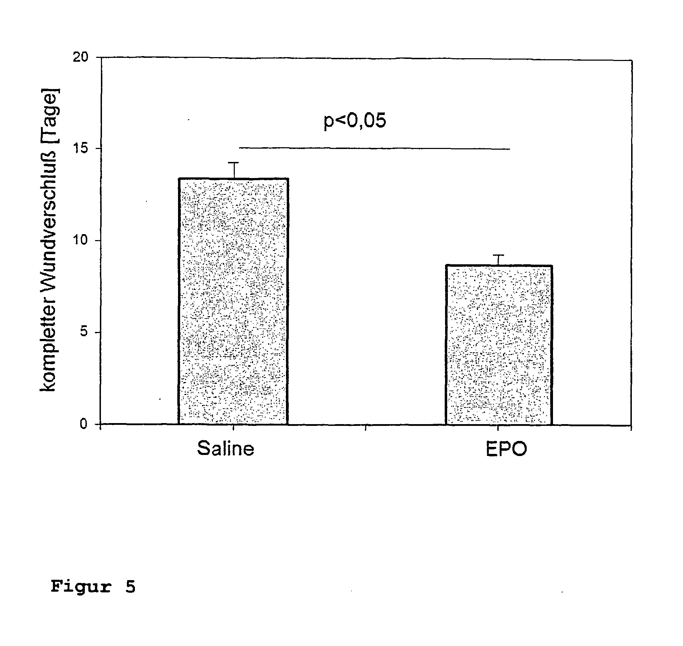

Figur 5 zeigt die Wirkung von Erythropoietin auf die Wundheilung. Die Figur zeigt, dass bei Behandlung einer standardisierten Hautwunde, die bei Mäusen mittels einer Gewebestanze gesetzt worden war, mit Erythropoietin die Wunde bereits nach sieben bis acht Tagen vollständig verschlossen war, während bei Behandlung der Wunde mit physiologischer Kochsalzlösung (Saline) die Wunde erst nach dreizehn bis vierzehn Tagen vollständig verschlossen war. Die Behandlung mit Erythropoietin beziehungsweise physiologischer Kochsalzlösung begann 7 Tage vor Setzen der Hautwunde. Rekombinantes humanes Erythropoietin wurde einmalig wöchentlich mittels einer s.c. (subcutan)-Injektion (0,1 µg/kg Aranesp) verabreicht (je Gruppe n = 5).

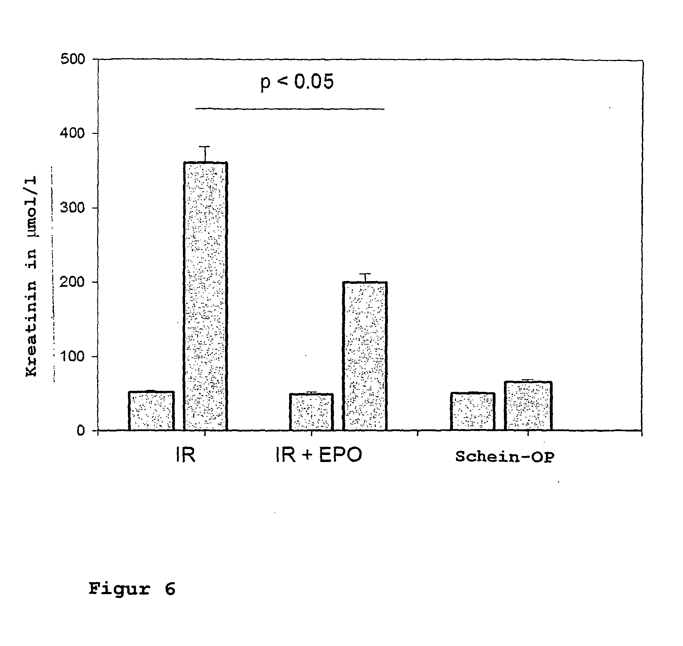

Figur 6 zeigt, dass Erythropoietin den Nierenfunktionsverlust nach akutem Nierenversagen (akute Niereninsuffizienz) vermindert. Sprague Dawley-Ratten (250-300 g) wurden in die Studie eingeschlossen.

Die Ratten wurden mit Ketamin (120 mg/kg) und Rompun (10 mg/kg) narkotisiert. Eine der Versuchsgruppen erhielt 0,1 µg/kg Körpergewicht Aranesp einmalig am Tag vor Induktion des akuten Nierenversagens. Als Vergleich diente eine Gruppe von Versuchstieren, denen jeweils zeitgleich Kochsalz s.c. injiziert wurde. Mittels Setzen einer Arterienklemme auf die rechte Nierenarterien wurde der Blutfluss in die Niere für 45 Minuten unterbrochen. In dieser Zeit wurde linksseitig eine Nephrektomie durchgeführt. Eine Scheinoperation wurde bei einer weiteren Kontrollgruppe durchgeführt. Hierbei wurde das Abdomen eröffnet, die linke Nierenarterie freipräpariert, jedoch die Blutversorgung nicht unterbrochen und die kontralaterale rechte Niere entnommen. Alle Tiere wurde für 60 min narkotisiert und 24 h nach Operation getötet. Die 45-minütige Ischämie mit nachfolgender Reperfusion der verbleibenden rechten Niere führte in den Kochsalz-behandelten Tieren zu einem massiven akuten Nierenfunktionsverlust. Dieser reflektiert sich in einem Serum-Kreatinin-Wert, der 24 h nach der Ischämie-Reperfusion 7-mal größer ist als der Wert vor der Ischämie-Reperfusion (p < 0,05). Demgegenüber zeigten die mit dem Erythropoietin-Analog Aranesp behandelten Tiere nur einen vierfachen Anstieg der Serum-Kreatinin-Werte einen Tag nach Induktion des Ischämie-Reperfusionsschadens. Bei den linksseitig nephrektomierten Tieren mit scheinoperierter rechter Niere kam es zu keinem Anstieg der Retentionswerte. Die Figur zeigt die Kreatinin-Konzentration im Serum von EPO-behandelten Tieren (IR + EPO), NaCl-behandelten Tieren (IR) und scheinoperierten Tieren (Schein-OP) vor Ischämie-Reperfusions (IR)-Verletzung und 24 Stunden danach. Aus der Figur geht hervor, dass die Serum-Kreatinin-Konzentration bei den Aranesp-behandelten Tieren 24 Stunden nach Ischämie-Reperfusions-Verletzung gegenüber der Kontrolle ohne (NaCl-Behandlung) fast halbiert ist.

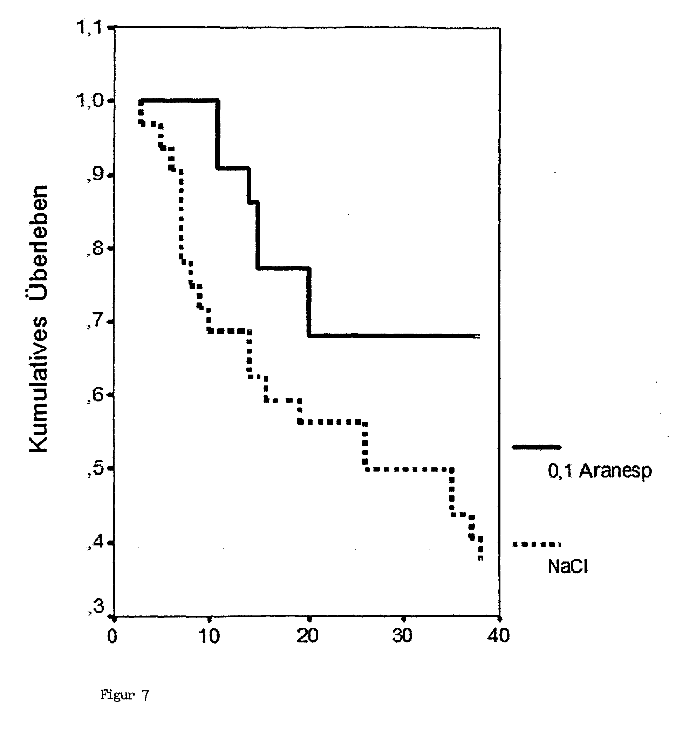

Figur 7 zeigt die Kaplan-Mayer-Überlebenskurven von zwei Versuchsgruppen nach Induktion einer chronischen Niereninsuffizienz, die entweder mit Aranesp oder NaCl behandelt wurden. 8 Wochen alte Spraque Dawley-Ratten wurden in die Studie eingeschlossen. Die Ratten wurden mit Ketamin (120 mg/kg) und Rompun (10 mg/kg) narkotisiert. Ihnen wurde am Tag 0 die rechte Niere entnommen, welche sofort nach Entnahme für eine histologische Untersuchung in Formalin fixiert wurde. Bei der linken Niere wurden die Segmentarterien, welche den oberen und unteren Nierenpol versorgen, ligiert. Dadurch kommt es zu einem Niereninfarkt der entsprechenden Nierenareale und nur das mittlere Nierendrittel bleibt funktionell erhalten. Einmal wöchentlich erhielten die Ratten Aranesp (0,1 µg/kg Körpergewicht) oder NaCl s.c. gespritzt. Die mit dem Erythropoietin-Analog Aranesp behandelten Tiere weisen einen signifikanten Überlebensvorteil gegenüber den kochsalzbehandelten Tieren auf (p = 0,027;Log Rank-Test).

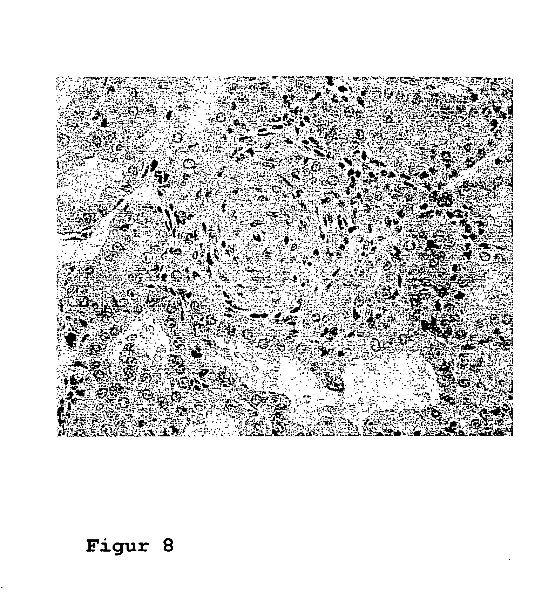

Die Figuren 8-15 zeigen lichtmikroskopische Nierenschnitte 6 Wochen nach Induktion einer chronischen Niereninsuffizienz von zwei Versuchsgruppen, die entweder mit Aranesp oder NaCl behandelt wurden und deren Kaplan-Mayer-Überlebenskurven in Figur 7 dargestellt sind.

Figur 8 zeigt die histologischen Veränderungen bei einer Spraque-Dawley-Ratte mit chronischer Niereninsuffizienz nach jeweils einmal wöchentlicher NaCl-Behandlung beginnend direkt nach Induktion der chronischen Niereninsuffizienz für einen Zeitraum von 6 Wochen. Die chronische Niereninsuffizienz resultiert aus der Entnahme der rechten Niere und der Ligierung der Segmentarterien, die den oberen und unteren Nierenpol versorgen, der linken Niere. Die Figur zeigt eine mittelgroße präglomeruläre Arterie mit charakteristischer zwiebelschalenartiger Gefäßwand-Proliferation bei schwerer hypertensiver Schädigung, einer sogenannten malignen Nephrosklerose mit Endarteriitis Fahr.

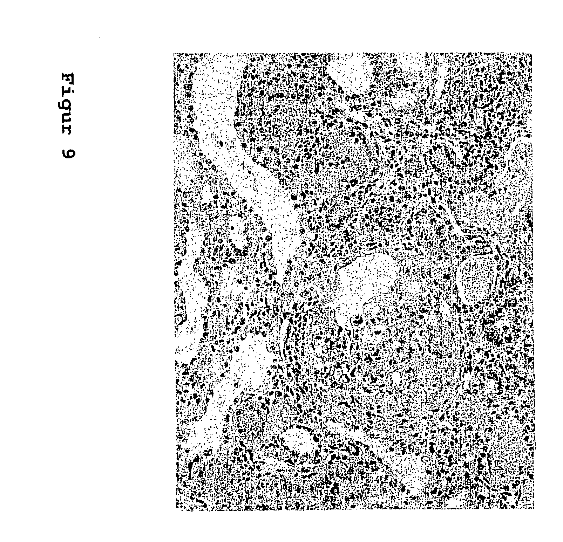

Figur 9 zeigt die histologischen Veränderungen bei einer Spraque-Dawley-Ratte mit chronischer Niereninsuffizienz nach jeweils einmal wöchentlicher NaCl-Behandlung beginnend direkt nach Induktion der chronischen Niereninsuffizienz für einen Zeitraum von 6 Wochen. Die chronische Niereninsuffizienz resultiert aus der Entnahme der rechten Niere und der Ligierung der Segmentarterien, die den oberen und unteren Nierenpol versorgen, der linken Niere. Die Figur zeigt floride fokal-segmentale Glomerulosklerose als sogenannte proliferative FSGS (Glomerulum rechts). Das andere Glomerulum (links) zeigt ischämischen Kollaps des Schlingenkonvolutes. Am unteren des Bildes ist ein kleines Gefäß mit schwerem Endothelschaden zu sehen. Die beobachteten histologischen Veränderungen entsprechen einem hypertensiven Organschaden beziehungsweise Veränderungen im Rahmen einer Überlastungsnephropatie nach 5/6 Nephrektomie.

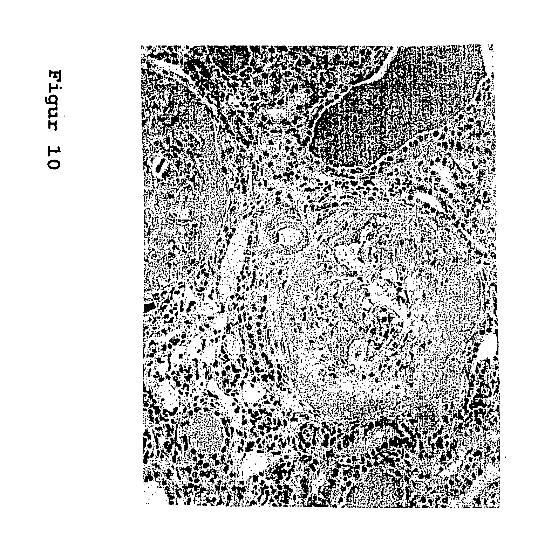

Figur 10 zeigt die histologischen Veränderungen bei einer Spraque-Dawley-Ratte mit chronischer Niereninsuffizienz nach jeweils einmal wöchentlicher NaCl-Behandlung beginnend direkt nach Induktion der chronischen Niereninsuffizienz für einen Zeitraum von 6 Wochen. Die chronische Niereninsuffizienz resultiert aus der Entnahme der rechten Niere und der Ligierung der Segmentarterien, die den oberen und unteren Nierenpol versorgen, der linken Niere. Die Figur zeigt eine nahezu vollständige Sklerose beziehungsweise Destruktion eines kompensatorisch vergrößerten Glomerulums mit ausgeprägter Hyalinose beziehungsweise fibrinoider Nekrose der dazugehörigen afferenten Arteriole.

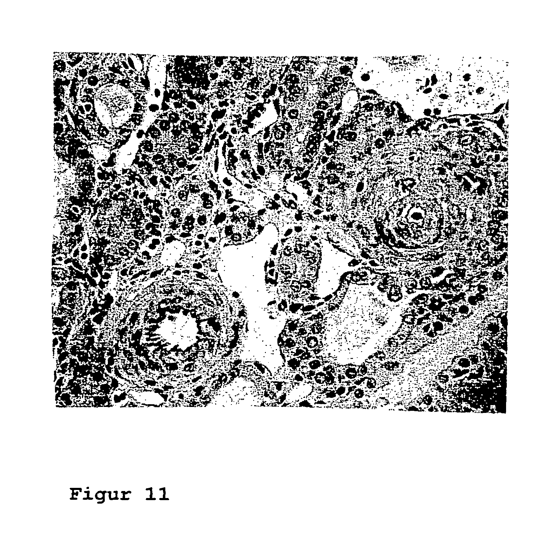

Figur 11 zeigt die histologischen Veränderungen bei einer Spraque-Dawley-Ratte mit chronischer Niereninsuffizienz nach jeweils einmal wöchentlicher NaCl-Behandlung beginnend direkt nach Induktion der chronischen Niereninsuffizienz für einen Zeitraum von 6 Wochen. Die chronische Niereninsuffizienz resultiert aus der Entnahme der rechten Niere und der Ligierung der Segmentarterien, die den oberen und unteren Nierenpol versorgen, der linken Niere. Die Figur zeigt eine kleine präglomeruläre Arterie mit charakteristischer zwiebelschalenartiger Gefäßwandproliferation und Wandnekrose bei schwerer hypertensiver Schädigung, einer sogenannten malignen Nephrosklerose (vergleiche Bild rechts). Links ist eine regelrechte (noch) nicht geschädigte Arteriole zu sehen.

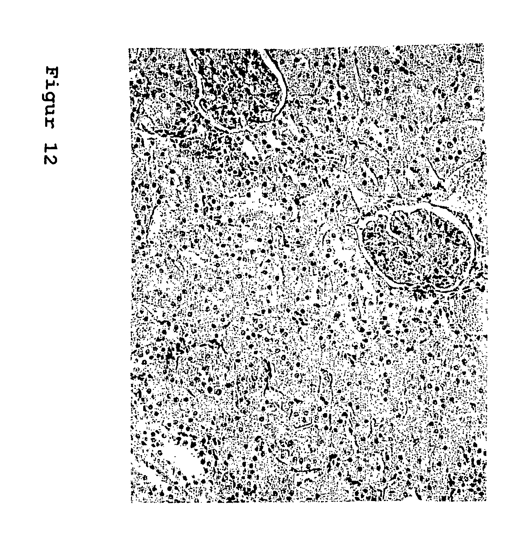

Figur 12 zeigt die histologischen Veränderungen bei einer Spraque-Dawley-Ratte mit chronischer Niereninsuffizienz nach jeweils einmal wöchentlicher Aranesp (EPO)-Behandlung (0,1 µg/kg Aranesp) beginnend direkt nach Induktion der chronischen Niereninsuffizienz für einen Zeitraum von 6 Wochen. Die chronische Niereninsuffizienz resultiert aus der Entnahme der rechten Niere und der Ligierung der Segmentarterien, die den oberen und unteren Nierenpol versorgen, der linken Niere. Die Figur zeigt ein regelrechtes Glomerulum mit zartem afferentem Gefäß. Im Tubulointerstitium ist kein pathologischer Befund festzustellen.

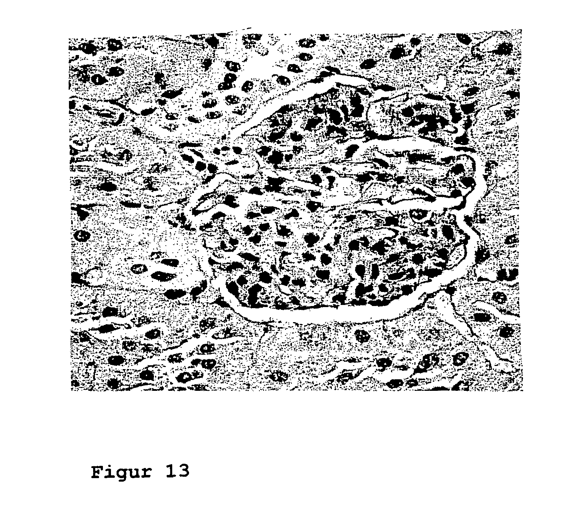

Figur 13 zeigt die histologischen Veränderungen bei einer Spraque-Dawley-Ratte mit chronischer Niereninsuffizienz nach jeweils einmal wöchentlicher Aranesp (EPO)-Behandlung (0,1 µg/kg Aranesp) beginnend direkt nach Induktion der chronischen Niereninsuffizienz für einen Zeitraum von 6 Wochen. Die chronische Niereninsuffizienz resultiert aus der Entnahme der rechten Niere und der Ligierung der Segmentarterien, die den oberen und unteren Nierenpol versorgen, der linken Niere. Die Figur zeigt ein regelrechtes Glomerulum mit zartem afferentem Gefäß (630-fache Vergrößerung). Im Tubolointerstitium ist kein pathologischer Befund festzustellen.

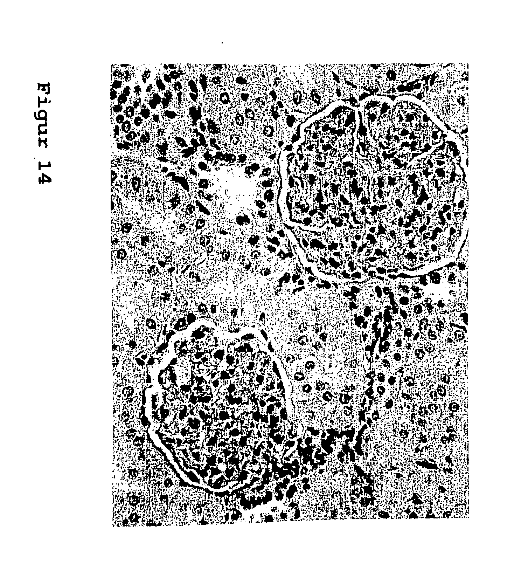

Figur 14 zeigt die histologischen Veränderungen bei einer Spraque-Dawley-Ratte mit chronischer Niereninsuffizienz nach jeweils einmal wöchentlicher Aranesp (EPO)-Behandlung (0,1 µg/kg Aranesp) beginnend direkt nach Induktion der chronischen Niereninsuffizienz für einen Zeitraum von 6 Wochen. Die chronische Niereninsuffizienz resultiert aus der Entnahme der rechten Niere und der Ligierung der Segmentarterien, die den oberen und unteren Nierenpol versorgen, der linken Niere. Die Figur zeigt ein regelrechtes Glomerulum mit zartem afferentem Gefäß. Im Tubolointerstitium ist kein pathologischer Befund festzustellen.

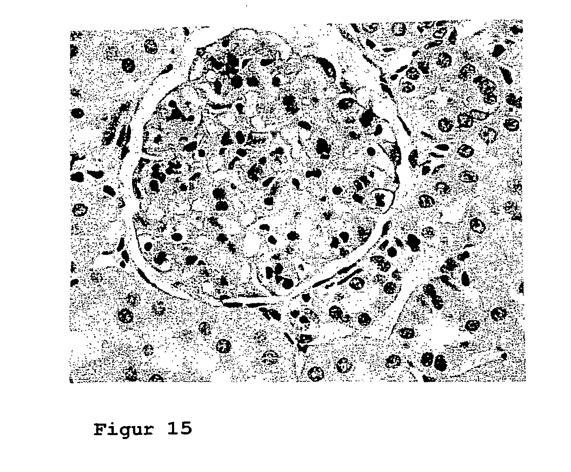

Figur 15 zeigt die histologischen Veränderungen bei einer Spraque-Dawley-Ratte mit chronischer Niereninsuffizienz nach jeweils einmal wöchentlicher Aranesp (EPO)-Behandlung (0,1 µg/kg Aranesp) beginnend direkt nach Induktion der chronischen Niereninsuffizienz für einen Zeitraum von 6 Wochen. Die chronische Niereninsuffizienz resultiert aus der Entnahme der rechten Niere und der Ligierung der Segmentarterien, die den oberen und unteren Nierenpol versorgen, der linken Niere. Die Figur zeigt ein regelrechtes Glomerulum mit zartem afferentem Gefäß (630-fache Vergrößerung). Im Tubolointerstitium ist kein pathologischer Befund festzustellen.

### Vergleichsbeispiel 1

### Wirkung von EPO bei Patienten mit renaler Anämie

Die Wirkung von Erythropoietin bei Patienten mit renaler Anämie (Hb < 10,5 g/dl) als Folge von Nierenkrankheit im Endstadium (präterminale Niereninsuffizienz; Creatinin-Clearance < 35 ml/min) wurde untersucht. 11 Patienten wurden über einen Zeitraum von mindestens 8 Wochen mit Erythropoietin in wöchentlichen Dosen von durchschnittlich 5000 IU rhEPO (rekombinantes humanes Erythropoietin) intravenös beziehungsweise subkutan behandelt. Nach Erythropoietin-Behandlung wurden die endothelialen Vorläuferzellen im Blut der Patienten über einen Zeitraum von 20 Wochen untersucht, wobei nach 0, 2, 4, 6 und 8 Wochen endotheliale Vorläuferzellen bezüglich ihrer Anzahl als auch ihres Differenzierungsstatus mittels Durchflusszytometrie und eines Kulturtestes analysiert wurden.

Bei zirkulierenden peripheren Blutstammzellen (CPBSC) handelt es sich um eine kleine Zellpopulation, die sowohl das CD34-Antigen als auch das CD45-Antigen exprimieren. Auf der Basis der ISHAGE-Richtlinien wurde ein Test zur Bestimmung der Anzahl von CPBSC mittels Durchflusszytometrie entwickelt (Sutherland et al., J. Hematother., 5 (1996), 213-226). Unter Verwendung dieses Tests wurden sowohl dies Expressionsmuster von CD34- und CD45-Zellen als auch die Morphologie der Stammzellen bestimmt. Auf diese Weise wurde sowohl die absolute Anzahl von CPBSC pro µl als auch der prozentuale Anteil von CPBSC an der Gesamtleukozytenzahl bestimmt.

Figur 1 zeigt die Ergebnisse einer FACS-Analyse zirkulierender CD34⁺-Stammzellen auf der Basis der ISHAGE-Richtlinien.

Figur 2 zeigt die Anzahl der mittels FACS-Analyse ermittelten CD34⁺-Stammzellen über einen Zeitraum von 8 Wochen.

### Zellkultur-Test

Mononucleäre periphere Blutzellen (PBMCs) wurden mittels Dichtezentrifugation über Ficoll aus menschlichen Blutproben entsprechend dem in Asahara, Science, 275 (1997), 964-967, beschriebenen Verfahren isoliert. Die Zellen wurden auf Kulturplatten mit Fibronectin ausplattiert und in EC-Basalmedium gehalten. EC-Basalmedium besteht aus EBM-2-Basalmedium (Fa. Clonetics) und EGM-2 Quots (hEGF; GA-1000 (Gentamicin, Amphotericin-B) FBS, VEGF, hFGF-B (w/heparin), R³-IGF-1, Ascorbic Acid, Heparin). Nach 4-tägiger Kultivierung wurden nichtadhärente Zellen durch Waschen der Platten entfernt. Die verbleibenden adhärenten Zellen wurden mit Trypsin behandelt und erneut ausgesät. Anschließend wurden sie für weitere 3 Tage kultiviert. Zellen mit endothelialem Phänotyp wurden durch Positivfärbung bezüglich zwei unterschiedlicher Endothelialmarker am Tag 7 nach Isolation identifiziert. Dabei handelt es sich um DiImarkiertes acetyliertes Lipoprotein geringer Dichte (acLDL-DiI) und Ulex europaeus-Aglutinin-1 (UEA-1). Die Ergebnisse dieser Untersuchung sind in Figur 3 dargestellt.

Die Ergebnisse zeigen, dass Erythropoietin endotheliale Vorläuferzellen mobilisieren und die Anzahl zirkulierender endothelialer Vorläuferzellen erhöhen kann. Dabei werden funktionelle Defizite, die unter bestimmten pathologischen Zuständen wie renaler Anämie auftreten, ausgeglichen. Diese Ergebnisse sind in Figur 4 dargestellt.

Mittels Durchflusszytometrie wurde ermittelt, dass bei Patienten mit Nierenkrankheit im Endstadium die Anzahl zirkulierender CD34⁺-Stammzellen der Anzahl zirkulierender CD34⁺-Stammzellen im Blut von gesunden Probanden entspricht. Nach Beginn der Erythropoietin-Behandlung erhöht sich die Anzahl CD34⁺-Stammzellen im Blutkreislauf signifikant um mehr als 50 %. Unter Verwendung des Zellkulturtestes wurde bestimmt, dass nach Behandlung mit Erythropoietin die Anzahl der Zellen, die einen endothelialen Phänotyp entwickeln, dramatisch ansteigt. In einem funktionellen Zellkulturtest erhöhte sich die stark beeinträchtigte Fähigkeit von endothelialen Vorläuferzellen um das mehr als 3-fache.

### Beispiel 2

### Verbesserte Wundheilung durch den systemischen Einsatz von rhEPO

FVB/N-Mäuse wurden durch Inhalationsanästhesie mit Isofloran narkotisiert. Die Behaarung der beiden Hinterläufe wurde mit Hilfe einer Enthaarungslotion entfernt und mit 70%igen Alkohol desinfiziert. Mittels einer sterilen, 4 mm-Einweg-Biopsiegewebestanze wurde jeweils eine Hautwunde auf der rechten Flanke der Mäuse gesetzt. Die gegenüberliegende Seite diente als interne Kontrolle. Einmalig wurde eine postoperative antibiotische Abschirmung mit Penicillin G (20000 Einheiten/kg) durchgeführt. Über den gesamten Untersuchungszeitraum erfolgten einmalig wöchentliche subkutane Injektionen von rekombinantem humanen Erythropoietin-Analog Aranesp (0,1 µg/kg Körpergewicht) über den gesamten Studienzeitraum. Die Behandlung begann sieben Tage vor Entnahme der Gewebestanze. Die Ergebnisse sind in Figur 5 dargestellt. Sie zeigen, dass die Verabreichung von EPO den Wundheilungsprozess erheblich beschleunigt.

### Beispiel 3

### Verminderung der Progression der chronischen Niereninsuffizienz durch Erythropoietin-Behandlung

Acht Wochen alte Spraque-Dawley-Ratten wurden mit Ketamin (120 mg/kg) und Rompun (10 mg/kg) narkotisiert. Den Ratten wurden am Tage 0 die rechte Niere entnommen, welche sofort nach Entnahme für eine histologische Untersuchung in Formalin fixiert wurde. Bei der linken Niere wurden die Segmentarterien, welche den oberen und unteren Nierenpol versorgen, ligiert. Dadurch kommt es zu einem Niereninfarkt der entsprechenden Nierenareale, wobei nur das mittlere Nierendrittel funktionell erhalten bleibt. Einmal wöchentlich erhielten die Ratten das Erythropoietin-Analog Aranesp in einer Dosis von 0,1 µg/kg Körpergewicht oder zur Kontrolle NaCl subkutan (s.c.) gespritzt.

Figur 7 zeigt die Kaplan-Mayer-Überlebenskurve beider Versuchsgruppen. Die Aranesp-behandelten Tiere haben ein deutlich verbessertes Überleben verglichen mit den Kochsalz-behandelten Kontrolltieren.

In den Figuren 12-15 ist gezeigt, dass nach Behandlung mit Erythropoietin das Nierengewebe keine pathologischen Veränderungen zeigt, während nach Behandlung mit NaCl schwere pathologische Veränderungen sichtbar sind (vergleiche Figuren 8-11). Weitere histologische Untersuchungen ergaben, dass bei Aranesp-behandelten Tieren eine deutlich höhere Gefäßdichte (CD31) zu beobachten ist als bei Kochsalz-behandelten Tieren (Daten nicht gezeigt).

### Beispiel 4

### Verminderung der Progression akuter Niereninsuffizienz

Für diese Untersuchung wurden Spraque-Dawley-Ratten mit einem Körpergewicht von 250 bis 300 g eingesetzt. Eine der Versuchsgruppen erhielt einmalig am Tag vor Induktion des akuten Nierenversagens Aranesp in einer Dosis von 0,1 µg/kg Körpergewicht. Die Ratten wurden mit Ketamin (120 mg/kg Körpergewicht) und Rompun (10 mg/kg) narkotisiert. Als Vergleich diente eine Gruppe von Versuchstieren, denen jeweils zeitgleich Kochsalz s.c. injiziert wurde. Der Blutfluss in der Niere wurde für 45 Minuten unterbrochen, indem eine Arterienklemme auf die rechte Nierenarterie gesetzt wurde. In dieser Zeit wurde linksseitig eine Nephrektomie durchgeführt. Bei einer weiteren Kontrollgruppe wurde eine Scheinoperation durchgeführt. Hier wurde das Abdomen eröffnet, die linke Nierenarterie freipräpariert, jedoch die Blutversorgung nicht unterbrochen, und die kontralaterale rechte Niere entnommen. Alle Tiere wurden für einen Zeitraum von 60 Minuten narkotisiert und 24 Stunden nach Operation getötet.

Die 45-minütige Ischämie mit nachfolgender Reperfusion der verbleibenden rechten Niere führte in den kochsalzbehandelten Tieren zu einem massiven akuten Nierenfunktionsverlust. Dieser fand seinen Ausdruck in einem um den Faktor 7 angestiegenen Serumkreatinin-Wert (p < 0,05). Demgegenüber zeigten die mit dem Erythropoietin-Analog Aranesp behandelten Tiere nur einen vierfachen Anstieg des Serumkreatinin-Wertes einen Tag nach Induktion des Ischämie-Reperfusions-Schadens. Bei den linksseitig nephrektonierten Tieren mit scheinoperierter rechter Niere kam es zu keinem Anstieg der Retentionswerte. Die Ergebnisse sind in Figur 6 dargestellt.

## Patentansprüche

1. Verwendung von Erythropoietin zur Herstellung einer pharmazeutischen Zusammensetzung enthaltend eine subpolycythämische EPO-Dosis, die einer wöchentlichen Dosis von 1 bis 90 IU EPO/kg Körpergewicht entspricht, zur Behandlung von chronischer Niereninsuffizienz.

2. Verwendung von Erythropoietin zur Herstellung einer pharmazeutischen Zusammensetzung enthaltend eine subpolycythämische EPO-Dosis, die einer wöchentlichen Dosis von 1 bis 90 IU EPO/kg Körpergewicht entspricht, zur Behandlung von akuter Niereninsuffizienz.

3. Verwendung von Erythropoietin zur Herstellung einer pharmazeutischen Zusammensetzung enthaltend eine subpolycythämische EPO-Dosis, die einer wöchentlichen Dosis von 1 bis 90 IU EPO/kg Körpergewicht entspricht, zur Wundheilung.

4. Verwendung nach einem der Ansprüche 1 bis 3, wobei die pharmazeutische Zusammensetzung zur parenteralen, insbesondere intravenösen, intramuskulären, intrakutanen oder subkutanen Verabreichung geeignet ist.

5. Verwendung nach Anspruch 4, wobei die pharmazeutische Zusammensetzung als Injektion oder Infusion vorliegt.

6. Verwendung nach einem der Ansprüche 1 bis 5, wobei die pharmazeutische Zusammensetzung zur pulmonalen Verabreichung geeignet ist.

7. Verwendung nach Anspruch 6, wobei die pharmazeutische Zusammensetzung als wässrige Lösung, nicht-wässrige Lösung oder Pulver vorliegt.

8. Verwendung nach Anspruch 6 oder 7, wobei die pharmazeutische Zusammensetzung in Form eines Aerosol-Präparates vorliegt.

9. Verwendung nach einem der Ansprüche 1 bis 3, wobei die pharmazeutische Zusammensetzung zur oralen Verabreichung geeignet ist.

10. Verwendung nach Anspruch 9, wobei die pharmazeutische Zusammensetzung als Lösung, Suspension, Emulsion oder Tablette vorliegt.

11. Verwendung nach einem der Ansprüche 1 bis 10, wobei die pharmazeutische Zusammensetzung mindestens einen weiteren Wirkstoff zur Stimulation endothelialer Vorläuferzellen enthält.

12. Verwendung nach Anspruch 11, wobei es sich bei dem weiteren Wirkstoff um VEGF, PIGF, GM-CSF, einen HMG-CoA-Reduktase-Inhibitor und/oder einen NO-Donator insbesondere L-Arginin handelt.

13. Verwendung nach Anspruch 12, wobei der HMG-CoA-Reduktase-Inhibitor ein Statin wie Simvastatin, Mevastatin oder Atorvastatin ist.

14. Verwendung nach einem der Ansprüche 1 bis 13, wobei Erythropoietin menschliches oder tierisches Erythropoietin ist.

15. Verwendung nach Anspruch 14, wobei Erythropoietin aus menschlichem Urin, dem Urin oder Plasma von an aplastischer Anämie leidenden Patienten, Gewebekulturen von menschlichen Nierenkrebszellen, die Fähigkeit zur Bildung von menschlichem Erythropoietin aufweisenden menschlichen Lymphoblastenzellen oder einer durch Zellfusion einer menschlichen Zelllinie erhaltenen Hybridomkultur isoliert ist.

16. Verwendung nach Anspruch 14, wobei Erythropoietin ein mittels DNA-Rekombinationstechniken hergestelltes Erythropoietin ist.

## Claims

1. The use of erythropoietin for producing a pharmaceutical composition comprising a subpolycythemic EPO-dose corresponding to a weekly dose of from 1 to 90 IU EPO/kg body weight for the treatment of chronic renal insufficiency.

2. The use of erythropoietin for producing a pharmaceutical composition comprising a subpolycythemic EPO-dose corresponding to a weekly dose of from 1 to 90 IU EPO/kg body weight for the treatment of acute renal insufficiency.

3. The use of erythropoietin for producing a pharmaceutical composition comprising a subpolycythemic EPO-dose corresponding to a weekly dose of from 1 to 90 IU EPO/kg body weight for wound healing.

4. The use as claimed in any of claims 1 to 3, wherein the pharmaceutical composition is suitable for parenteral, in particular intravenous, intramuscular, intracutaneous or subcutaneous, administration.

5. The use as claimed in claim 4, wherein the pharmaceutical composition is in the form of an injection or infusion.

6. The use as claimed in any of claims 1 to 5, wherein the pharmaceutical composition is suitable for pulmonary administration.

7. The use as claimed in claim 6, wherein the pharmaceutical composition is in the form of an aqueous solution, non-aqueous solution or powder.

8. The use as claimed in any of claims 6 or 7, wherein the pharmaceutical composition is in the form of an aerosol preparation.

9. The use as claimed in any of claims 1 to 3, wherein the pharmaceutical composition is suitable for oral administration.

10. The use as claimed in claim 9, wherein the pharmaceutical composition is in the form of a solution, suspension, emulsion or tablet.

11. The use as claimed in any of claims 1 to 10, wherein the pharmaceutical composition comprises at least one further active ingredient to stimulate endothelial progenitor cells.

12. The use as claimed in claim 11, wherein the further active ingredient is VEGF, PIGF, GM-CSF, an HMG-CoA reductase inhibitor and/or an NO donor, especially L-arginine.

13. The use as claimed in claim 12, wherein the HMG-CoA reductase inhibitor is a statin such as simvastatin, mevastatin or atorvastatin.

14. The use as claimed in any of claims 1 to 13, wherein erythropoietin is human or animal erythropoietin.

15. The use as claimed in claim 14, wherein the erythropoietin is isolated from human urine, the urine or plasma of patients suffering from aplastic anemia, tissue cultures of human renal cancer cells, human lymphoblast cells having the ability to produce human erythropoietin, or a hybridoma culture obtained by cell fusion of a human cell line.

16. The use as claimed in claim 14, wherein erythropoietin is an erythropoietin produced by DNA recombination techniques.

## Revendications

1. Utilisation d'érythropoïétine en vue de la préparation d'une composition pharmaceutique contenant une dose d'EPO subpolycythémique qui correspond à une dose hebdomadaire de 1 à 90 UI d'EPO/kg de poids corporel, en vue du traitement d'une insuffisance rénale chronique.

2. Utilisation d'érythropoïétine en vue de la préparation d'une composition pharmaceutique contenant une dose d'EPO subpolycythémique qui correspond à une dose hebdomadaire de 1 à 90 UI d'EPO/kg de poids corporel, en vue du traitement d'une insuffisance rénale aiguë.

3. Utilisation d'érythropoïétine en vue de la préparation d'une composition pharmaceutique contenant une dose d'EPO subpolycythémique qui correspond à une dose hebdomadaire de 1 à 90 UI d'EPO/kg de poids corporel, en vue de la guérison de lésions.

4. Utilisation selon l'une quelconque des revendications 1 à 3, la composition pharmaceutique étant appropriée à l'administration par voie parentérale, notamment intraveineuse, intramusculaire, intracutanée ou sous-cutanée.

5. Utilisation selon la revendication 4, la composition pharmaceutique étant présente sous la forme d'une injection ou d'une infusion.

6. Utilisation selon l'une quelconque des revendications 1 à 5, la composition pharmaceutique étant appropriée à l'administration par voie pulmonaire.

7. Utilisation selon la revendication 6, la composition pharmaceutique étant présente sous la forme d'une solution aqueuse, d'une solution non aqueuse ou d'une poudre.

8. Utilisation selon la revendication 6 ou 7, la composition pharmaceutique étant présente sous la forme d'une préparation pour aérosol.

9. Utilisation selon l'une quelconque des revendications 1 à 3, la composition pharmaceutique étant appropriée à l'administration par voie orale.

10. Utilisation selon la revendication 9, la composition pharmaceutique étant présente sous la forme d'une solution, suspension, émulsion ou de comprimés.

11. Utilisation selon l'une quelconque des revendications 1 à 10, la composition pharmaceutique contenant au moins un autre ingrédient actif en vue de la stimulation de cellules précurseurs endothéliales.

12. Utilisation selon la revendication 11, l'autre ingrédient actif étant VEGF, PIGF, GM-CSF, un inhibiteur de HMG-CoA-réductase et/ou un donneur de NO, notamment la L-arginine.

13. Utilisation selon la revendication 12, l'inhibiteur de HMG-CoA-réductase étant une statine comme la simvastatine, la mévastatine ou l'atorvastatine.

14. Utilisation selon l'une quelconque des revendications 1 à 13, l'érythropoïétine étant de l'érythropoïétine humaine ou animale.

15. Utilisation selon la revendication 14, l'érythropoïétine étant isolée à partir de l'urine humaine, de l'urine ou du plasma de patients souffrant d'anémie aplasique, de cultures de tissu de cellules cancéreuses rénales humaines, de cellules de lymphoblastes humains présentant la capacité de former de l'érythropoïétine humaine ou d'une culture d'hybridome obtenue par fusion cellulaire d'une lignée cellulaire humaine.

16. Utilisation selon la revendication 14, l'érythropoïétine étant une érythropoïétine préparée au moyen de techniques de recombinaison d'ADN.
